# EUROPEAN PATENT APPLICATION

(11) **EP 1 992 348 A1**
(43) Date of publication of application: **19.11.2008**
(21) Application number: 07737912.1
(22) Date of filing: 07.03.2007
(51) Int. Cl.: A61K 31/519, A61K 45/00, A61P 1/00, A61P 13/12, A61P 19/00, A61P 25/00, A61P 43/00, C07D 487/04

(54) **PHARMACEUTICAL COMBINATION**

(30) Priority: 08.03.2006 JP 2006062768
(71) Applicant: Takeda Pharmaceutical Company Limited, Osaka-shi, Osaka 541-0045 (JP)
(72) Inventor: NOTOYA, Kohei, Osaka-shi Osaka 532-8686 (JP); OKA, Masahiro, Osaka-shi Osaka 532-8686 (JP)
(74) Representative: Dossmann, Gérard
(86) International application number: PCT/JP2007/054398
(87) International publication number: WO 2007/102531

(57) **Abstract**

Disclosed is a pharmaceutical combination comprising a calcium receptor modulator and a bone resorption inhibitor, wherein the calcium receptor modulator comprises a compound represented by the formula (I): 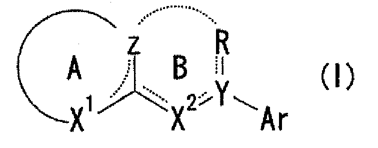 wherein the ring A represents a 5- to 7-membered ring which may be substituted; the ring B represents a 5- to 7-membered heterocyclic ring which may be substituted; X¹ represents CR¹ (wherein R¹ represents a hydrogen, a hydrocarbon group which may be substituted, or the like) or the like; X² represents N or the like; Y represents C or the like; Ar represents a cyclic group which may be substituted; R represents a hydrocarbon group which may be substituted, or the like; and ----- represents a single bond or a double bond; or a salt thereof or a prodrug of the compound or the salt.

## Description

### Technical Field

The present invention relates to a combined drug of calcium-sensing receptor (CaSR, hereinafter simply referred to as Ca receptor) modulator (agonist or antagonist).

### Background Art

Calcium ion (hereinafter, simply referred to as Calcium) plays an essential role to maintain and modulate functions of various cells such as endocrine and exocrine cells, etc., in addition to nerve and muscle. For this reason, the blood Ca level is strictly maintained in a narrow range. Parathyroid hormone (PTH) plays a central role in maintaining this blood Ca level. Therefore, secretion of PTH from parathyroid gland responds sharply to change in the blood Ca level and is must be modulated according to this. In fact, when the blood Ca level is changed, the blood PTH level is rapidly changed in response to this. The possibility of a mechanism by which the extracellular Ca concentration is sensed by parathyroid gland cells and the information transmitted into cells has been pointed out early by Brown et al. In 1993, they succeeded in the cloning and characterization of a Ca-sensing receptor (CaSR; hereinafter, simply referred to as Ca receptor) from bovine parathyroid (Nature, 366, 575-580(1993)).

The Ca receptor is composed of a large terminal extracellular region spanning 600 amino acids at the N-terminal, having seven transmembrane spanning domains like other G protein coupled receptors, and an intracellular region consisting of 200 or less amino acids at the caboxyl C-terminal.

It is considered that, when the extracellular Ca concentration is increased, phospholipase (PL)-C is activated, leading to increase in the intracellular Ca concentration and inhibition of PTH secretion due to increase in inositol triphosphate (IP₃). Since when a high value of the extracellular Ca concentration is maintained, the intracellular Ca concentration is thereafter increased continuously, it is considered that influx of Ca from the outside of a cell is also promoted. PL-A₂ and D are activated due to increase in extracellular Ca, but there is a possibility that these are via protein kinase (PK)-C and the like which are activated at the same time via Ca receptor. The Ca receptor also inhibits adenylyl cyclase via Gi protein or via arachidonic acid production due to activation of PL-A₂ and decreases intracellular cyclic AMP (Bone, 20, 303-309 (1997)).

Ca receptor mRNA is expressed in many tissues, and the expression amount is high, in parathyroid gland, thyroid gland C cell, medulla and cortex thick ascending limb (MTAL and CTAL) of kidney uriniferous tubule, intramedullary collecting tubule (IMCD) and encephalic subfornical organ (SFO) and hippocampus (Bone, 20, 303-309 (1997)). In addition, expression is recognized in many tissues such as encephalic hypothalamus, cerebellum and olfactory nucleus, regions other than TAL of renal uriniferous tubule, lung, stomach, pancreas, intestine and skin. Since the Ca receptor is present in various tissues, its physiological function has yet to be fully understood. However, it is expected that the Ca receptor modulating (agonistic or antagonistic) drug would provide for a novel treatment of various disease states which include the following:

### 1. Drugs for Treating Bone Diseases

Since the anabolic activity is manifested by intermittent administration of PTH, Ca receptor modulating drugs which are considered to be able to regulate secretion of PTH are promising as a drug for treating osteoporosis. In addition, Ca receptor modulating drugs which are selectable for thyroid gland C cell may be also effective for treating osteoporosis by stimulation of calcitonin secretion. Whether the same Ca receptor as that of parathyroid gland is present in osteoblast, osteoclast and bone cell or not is disputable. However, some Ca-sensing mechanism is assuredly present therein and, therefore, drugs which directly act on them can be expected as a drug for treating bone diseases.

### 2. Kidney-Acting Drugs

Handling of water and mineral in kidney is not only based on the results of function as a target organ for hormones, such as PTH, vitamin D etc., but also the Ca receptor in kidney is presumed to function in a response to the Ca concentration and the magnesium ion concentration in the extracellular fluid (Kidney Int, 50, 2129-2139 (1996)). Further, it is also considered that Ca receptor modulating drugs may modulate the blood amount in kidney, the amount of glomerulus filtration, renin secretion and activation of vitamin D in addition to control of influx and efflux of water and mineral.

### 3. Central Nervous System and Endocrine-Acting Drugs

Ca receptor is present in almost all areas in the central nervous system, and is remarkably expressed, in particular, in the hippocampus, cerebellum and subfornical organ (Brain Res, 744. 47-56 (1997)). Although the details of the function are still unclear, the term of Ca receptor expression after birth in the hippocampus is consistent with the term of acquisition of LTP (Long Tightening Phenomenon) (Develop Brain Res, 100, 13-21 (1997)) and, therefore, the relationship with memory and learning can be presumed. Therefore, Ca receptor modulating drugs which are high in brain-blood barrier permeability and selective for the central nerve system may be utilized for treating Alzheimer's disease. In addition, since dry mouth occurs in hypercalcemic patient, Ca receptor modulating drugs may control them. The presence of Ca receptor in mouse pituitary gland cells which secreteACTH has been reported (Mol Endocrinol, 10, 555-565 (1996)). It is also considered that Ca receptor modulating drugs can be applied to Sheehann's syndrome and hypopituitarism or hyperpituitarism.

### 4. Digestive System-Acting Drugs

It is considered that a Ca receptor is present in the Auerbach nerve plexus of the digestive tract and controls intestinal tract motion. Constipation is known in hypercalcemic patients and stimulation of digestive tract motion is known in hypocalcemic patients in clinical tests. The existence of a Ca receptor in the gastrin secreting cell (G cell) of the stomach has been reported (J. Clin Invest, 99, 2328-2333 (1997)), and intestinal tract absorption, constipation, diarrhea, defecation and secretion of acid in the stomach may be controlled by drugs which act on a Ca receptor in the digestive tract. Further, it has been found that a Ca receptor is present in human colon cancer cell strains and it controls c-myc expression and proliferation (Biochem Biophys Res Commum, 232, 80-83 (1997)), this is better consistent with the fact that the Ca uptake and sideration of colon and rectum cancers exhibit the negative correlation and, therefore, Ca receptor regulating drugs can be expected also as a drug for preventing and treating such cancers.

In addition, as a combined drug of Ca receptor antagonist, JP 2004-519428A discloses a therapeutic method for diseases or disorders characterized by abnormal bone or mineral homeostasis which comprises administering both a compound represented by the formula 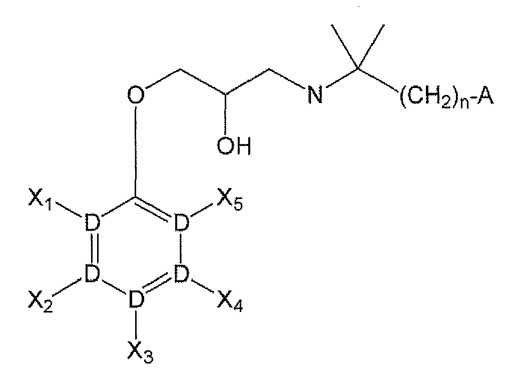 wherein, A is an aryl or fused aryl, dihydro or tetrahydro fused aryl, heteroaryl or fused heteroaryl, dihydro or tetrahydro fused heteroaryl, unsubstituted or substituted with any substituent being selected from the group consisting of OH, halogen, C₁₋₄ alkyl, C₁₋₄ alkoxy, C₃₋₆ cycloalkyl, CF₃, OCF₃, CN, and NO₂;
D is C or N with 1-2 N in ring, provided that X₁-X₅ are not present when D is N;
X₁ and X₅ are, independently, selected from the group consisting of H, halogen, CN, and NO₂, provided that either X₁ or X₅ is H; further provided that X₁ and X₅ are not present when D is N;
X₂, X₃ and X₄ are selected from the group consisting of H, halogen, O-C₁₋₄ alkyl, and J-K, wherein:
J is a covalent bond, alkylene, O-alkylene or alkenylene;
and K is selected from the group consisting of, CO₂R₅, CONR₄R'₄, OH, NR₄R'₄ and CN and provided X₂, X₃ and X₄ are not present when D is N;
R₄ and R₄' are independently H, alkyl, aryl or heteroaryl;
R₅ is H, alkyl, alkyl-(O-alkyl)ₘ-O-alkyl, aryl or heteroaryl:
   n is an integer from 0 to 4; and,
   m is an integer from 1-3;
and an anti-resorptive agent (estrogen, 1,25(OH)₂vitamin D3, calcitonin, selective estrogen receptor modulators, vitranectin receptor antagonists, V-H+-ATPase inhibitors, src SH2 antagonists, bisphosphonates and cathepsin K inhibitors).

### Disclosure of Invention

The object of the present invention is to provide a pharmaceutical combination comprising a calcium receptor modulator and a bone resorption inhibitor, wherein the calcium receptor modulator comprises a compound represented by the formula (II): 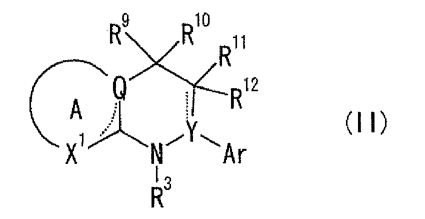 wherein ring A is an optionally substituted 5- to 7-membered ring;
Q is C, CR⁵ (wherein R⁵ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- is -CO-, -CS-, - SO- or -SO₂-, and Z² is an optionally substituted hydrocarbon group, an optionally substituted heterocyclic group, an optionally substituted hydroxyl group, or an optionally substituted amino group)), or N;
X¹ is CR¹ (wherein R¹ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above)), CR¹R² (wherein R¹ is as defined above, and R² is a hydrogen, or an optionally substituted hydrocarbon group), N, or NR¹³ (wherein R¹³ is a hydrogen, an optionally substituted hydrocarbon group, an optionally substituted hydroxyl group, an optionally substituted amino group, an optionally substituted heterocyclic group, or a group of the formula: -Z¹-Z² (wherein -Z¹- and Z² are as defined above));
R³ is a hydrogen, an optionally substituted hydrocarbon group, an optionally substituted hydroxyl group, an optionally substituted amino group, an optionally substituted heterocyclic group, or a group of the formula: -Z¹-Z² (wherein -Z¹- and Z² are as defined above);
Y is C, CR⁴ (wherein R⁴ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above)), or N;
Ar is an optionally substituted cyclic group;
R⁹ and R¹⁰ are the same or different and are a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and are as defined above); and
R¹¹ and R¹² are the same or different and are a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z^{1'}-Z² (wherein -Z^{1'}- is -CS-, -SO- or -SO₂-, and Z² is as defined above); or R⁹ and R¹⁰, or R¹¹ and R¹² may be combined to form an oxo group, methylene group or a ring; or R¹⁰ and R¹¹ may be combined to form a ring; and
----- is a single bond or a double bond; provided that
(1) when ring A is a 6-membered ring and Q is C or CR⁵, X¹ is C-Z¹-Z², C(-Z¹-Z²)R³ or N-Z¹-Z², and neither R⁹ nor R¹⁰ is a hydrogen, or R⁹ and R¹⁰ are not combined to form an oxo group, or R¹⁰ and R¹¹ are not combined to form a 5-membered ring,
(2) when ring A is a 6-membered ring and Q is N, X¹ is C-Z¹-Z², C(-Z¹-Z²)R² or N-Z¹-Z², and R⁹ and R¹⁰ are not combined to form an oxo group,
(3) when ring A is a 5-membered ring and Q is C or CR⁵, X¹ is C-Z¹-Z², C(-Z¹-Z²)R² or N-Z¹-Z², and Z² is an optionally substituted amino group, and
(4) when ring A is a 5-membered ring and Q is N, at least one of R⁹ and R¹⁰ is CHR¹⁵R¹⁶ (wherein at least one of R¹⁵ and R¹⁶ are the same or different and are a hydrogen, an optionally substituted hydrocarbon group, an optionally substituted hydroxyl group, an optionally substituted amino group, an optionally substituted thiol group, a halogen atom, an optionally substituted heterocyclic group, or a group of the formula: Z¹-Z² (wherein -Z¹- and Z² are as defined above)) and the other is other than an optionally substituted phenyl group; or a salt thereof or a prodrug thereof.

The present inventors have intensively investigated compounds having Ca receptor modulating activity, and as the result, have found a pharmaceutical combination of compounds represented by formulas (I), (II), (III) and (IIIa), and a bone resorption inhibitor as mentioned below.

That is, the present invention provides:
[1] A drug comprising a combination of a calcium receptor modulator and a bone resorption inhibitor, wherein the calcium receptor modulator comprises a compound represented by the formula (I): 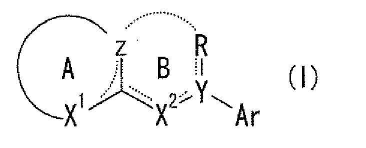 wherein ring A is an optionally substituted 5- to 7-membered ring;
   ring B is an optionally substituted 5- to 7-membered heterocyclic ring;
   X¹ is CR¹ (wherein R¹ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- is -CHO-, -CS-, - SO- or -SO₂-, and Z² is an optionally substituted hydrocarbon group, an optionally substituted heterocyclic group, an optionally substituted hydroxyl group, or an optionally substituted amino group)), CR¹R² (wherein R¹ is as defined above, R² is a hydrogen or an optionally substituted hydrocarbon group), N or NR¹³ (wherein R¹³ is a hydrogen, an optionally substituted hydrocarbon group, an optionally substituted hydroxyl group, an optionally substituted amino group, cyano group, a halogen atom, an optionally substituted heterocyclic group, or a group of the formula: -Z¹-Z² (wherein -Z¹- and Z² are as defined above));
   X² is N or NR³ (wherein R³ is a hydrogen, an optionally substituted hydrocarbon group, an optionally substituted hydroxyl group, an optionally substituted amino group, cyano group, a halogen atom, an optionally substituted heterocyclic group, or a group of the formula: -Z¹-Z² (wherein -Z¹- and Z² are as defined above);
   Y is C, CR⁴ (wherein R⁴ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula : -Z¹-Z² (wherein -Z¹- and Z² are as defined above)) or N;
   Z is CR⁵ (wherein R⁵ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹- Z² (wherein -Z¹- and Z² are as defined above)), CR⁵R⁶ (wherein, R⁵ is as defined above and
   R⁶ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above), and R⁵ and R⁶ may be the same or different), N or NR⁷ (wherein R⁷ is a hydrogen, an optionally substituted hydrocarbon group, an optionally substituted hydroxyl group, an optionally substituted amino group, cyano group, a halogen atom, an optionally substituted heterocyclic group, or a group of the formula: -Z¹-Z² (wherein -Z¹- and Z² are as defined above));
   Ar is an optionally substituted cyclic group;
   R is a hydrogen, an optionally substituted hydrocarbon group, an optionally substituted hydroxyl group, an optionally substituted amino group, an optionally substituted thiol group, an optionally substituted sulfonyl group or an optionally substituted sulfinyl group, or R and Z may be combined to form ring B; and
   ----- is a single bond or a double bond;
   or a salt thereof or a prodrug thereof;
[2] A drug comprising a combination of a calcium receptor modulator and a bone resorption inhibitor, wherein the calcium receptor modulator comprises a compound represented by the formula (II): 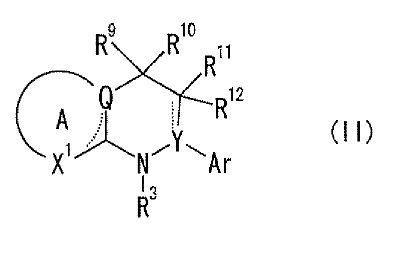 wherein ring A is an optionally substituted 5- to 7-membered ring;
   Q is C, CR⁵ (wherein, R⁵ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- is -CO-, -CS-, - SO- or -SO₂-, and Z² is an optionally substituted hydrocarbon group, an optionally substituted heterocyclic group, an optionally substituted hydroxyl group, or an optionally substituted amino group)), or N;
   X¹ is CR¹ (wherein R¹ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above)), CR¹R² (wherein R¹ is as defined above, and
   R² is a hydrogen, or an optionally substituted hydrocarbon group), N, or NR¹³ (wherein R¹³ is a hydrogen, an optionally substituted hydrocarbon group, an optionally substituted hydroxyl group, an optionally substituted amino group, an optionally substituted heterocyclic group, or a group of the formula: -Z¹-Z² (wherein -Z¹- and Z² are as defined above));
   R³ is a hydrogen, an optionally substituted hydrocarbon group, an optionally substituted hydroxyl group, an optionally substituted amino group, an optionally substituted heterocyclic group, or a group of the formula: -Z¹-Z² (wherein -Z¹- and Z² are as defined above);
   Y is C, CR⁴ (wherein R⁴ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above)), or N;
   Ar is an optionally substituted cyclic group;
   R⁹ and R¹⁰ are the same or different and are a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above); and
   R¹¹ and R¹² are the same or different and are a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z^{1'}-Z² (wherein -Z^{1'}- is -CS-, -SO- or -SO₂-, and Z² is as defined above); or R⁹ and R¹⁰, or R¹¹ and R¹² may be combined to form an oxo group, methylene group or a ring; or R¹⁰ and R¹¹ may be combined to form a ring; and
   ----- is a single bond or a double bond;
   provided that
   (1) when ring A is a 6-membered ring and Q is C or CR⁵, X¹ is C-Z¹-Z², C(-Z¹-Z²) R² or N-Z¹-Z², and neither R³ nor R¹⁰ is a hydrogen, or R⁹ and R¹⁰ are not combined to form an oxo group, or R¹⁰ and R¹¹ are not combined to form a 5-membered ring,
   (2) when ring A is a 6-membered ring and Q is N, X¹ is C-Z¹- Z², C(-Z¹-Z²) R² or N-Z¹-Z², and R⁹ and R¹⁰ are not combined to form an oxo group,
   (3) when ring A is a 5-membered ring and Q is C or CR⁵, X¹ is C-Z¹-Z², C(-Z¹-Z²)R² or N-Z¹-Z², and Z² is an optionally substituted amino group, and
   (4) when ring A is a 5-membered ring and Q is N, at least one of R⁹ and R¹⁰ is CHR¹⁵R¹⁶ (wherein at least one of R¹⁵ and R¹⁶ are the same or different and are a hydrogen, an optionally substituted hydrocarbon group, an optionally substituted hydroxyl group, an optionally substituted amino group, an optionally substituted thiol group, a halogen atom, an optionally substituted heterocyclic group, or a group of the formula: Z¹-Z² (wherein -Z¹- and Z² are as defined above)) and the other is other than an optionally substituted phenyl group; or a salt thereof or a prodrug thereof;
[3] A drug comprising a combination of a calcium receptor modulator and a bone resorption inhibitor, wherein the calcium receptor modulator comprises a compound represented by the formula (III): 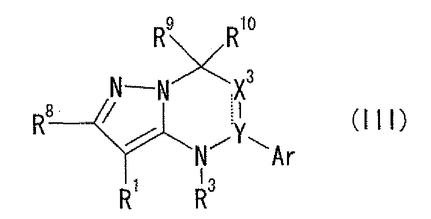 wherein R¹ is a hydrogen, an optionally substituted hydrocarbon group, an optionally substituted hydroxyl group, an optionally substituted thiol group, an optionally substituted amino group, cyano group, a halogen atom, an optionally substituted heterocyclic group, or a group of the formula: -Z¹-Z² (wherein -Z¹- is -CHO-, -CS-, -SO- or - SO₂- and Z² is an optionally substituted hydrocarbon group, an optionally substituted heterocyclic group, an optionally substituted hydroxyl group, or an optionally substituted amino group);
   R³ is a hydrogen, an optionally substituted hydrocarbon group, an optionally substituted hydroxyl group, an optionally substituted amino group, an optionally substituted heterocyclic group, or a group of the formula: -Z¹-Z² (wherein -Z¹- and Z² are as defined above);
   Y is C, CR⁴ (wherein R⁴ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above)) or N;
   R⁸ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above);
   Ar is an optionally substituted cyclic group;
   R⁹ and R¹⁰ are the same or different and are a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above), or R⁹ and R¹⁰ may be combined to form an oxo group, methylene group or a ring;
   X³ is a bond, oxygen atom, an optionally oxidized sulfur atom, N, NR^{7'} (wherein, R^{7'} is a hydrogen, an optionally substituted hydrocarbon group, an optionally substituted hydroxyl group, an optionally substituted amino group, an optionally substituted heterocyclic group, or a group of the formula -Z^{1'}-Z² (wherein -Z^{1'}- is -CS-, -SO- or -SO₂-, and Z² is as defined above)), or an optionally substituted bivalent C₁₋₂ hydrocarbon group; and
   ----- is a single bond or a double bond;
   provided that at least one of R⁹ and R¹⁰ is CHR¹⁵R¹⁶ (wherein, R¹⁵ and R¹⁶ are the same or different and are a hydrogen, an optionally substituted hydrocarbon group, an optionally substituted hydroxyl group, an optionally substituted amino group, an optionally substituted thiol group, a halogen atom, an optionally substituted heterocyclic group, or a group of the formula:-Z¹-Z² (wherein -Z¹- and Z² are as defined above)) and the other is other than an optionally substituted phenyl group; or a salt thereof or a prodrug thereof;
[4] A drug comprising a combination of a calcium receptor modulator and a bone resorption inhibitor, wherein the calcium receptor modulator comprises a compound represented by the formula (IIIa): 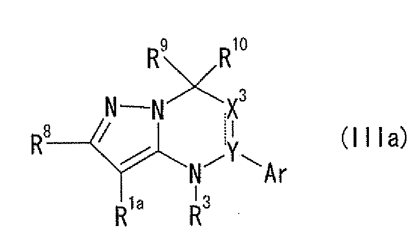 wherein, R^{1a} is (1) an optionally substituted heterocyclic group, or (2) a group of the formula: -Z^{1a}-Z^{2a} (wherein -Z^{1a}-is -CO-, -CS-, -SO- or -SO₂-, and Z^{2a} is (i) an optionally substituted heterocyclic group, (ii) -NR^{20a}(CR^{21a}R^{22a}R^{23a}) (wherein (a) R^{20a} is a hydrogen or an optionally substituted hydrocarbon group; and R^{21a} is an optionally substituted heterocyclic group which may be fused with an optionally substituted benzene ring, or an optionally substituted phenyl group which may be fused with an optionally substituted aromatic heterocyclic ring and R^{22a} and R^{23a} are the same or different and are an optionally substituted hydrocarbon group or an optionally substituted heterocyclic group or R^{22a} and R^{23a} may be combined to form a ring, or (b) R^{20a} is a hydrogen or an optionally substituted hydrocarbon group; and R^{21a}, R^{22a} and R^{23a} are the same or different and are an optionally substituted C₁₋₈ aliphatic hydrocarbon group, provided that the sum total of the carbon atoms is 7 or more), (iii) -NR^{20a}R^{25a} (wherein, R^{20a} is as defined above and R^{25a} is an optionally substituted C₆₋₁₀ aryl-C₂₋₄ alkyl, C₆₋₁₀ aryloxy-C₂₋₄ alkyl, C₆₋₁₀ arylamino-C₂₋₄ alkyl, C₇₋₁₄ aralkylamino-C₂₋₄ alkyl, heterocyclic ring-C₂₋₄ alkyl or heterocyclic group), (iv) a substituted 5- to 7-membered cyclic amino group, or (v) -OR^{24a} (wherein, R^{24a} is (a) an optionally substituted C₇₋₁₄ aralkyl group,
   (b) an optionally substituted C₃₋₇ alicyclic hydrocarbon group,
   (c) an optionally substituted C₇₋₂₄ aliphatic hydrocarbon group, or
   (d) an optionally substituted heterocyclic group);
   R³ is a hydrogen, an optionally substituted hydrocarbon group, an optionally substituted hydroxyl group, an optionally substituted amino group, an optionally substituted heterocyclic group, or a group of the formula: -Z¹-Z² (wherein -Z¹- is -CO-, -CS-, -SO- or -SO₂-, and Z² is an optionally substituted hydrocarbon group, an optionally substituted heterocyclic group, an optionally substituted hydroxyl group, or an optionally substituted amino group);
   Y is C, CR⁴ (wherein R⁴ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above)) or N;
   R⁸ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above);
   Ar is an optionally substituted cyclic group;
   R⁹ and R¹⁰ are the same or different and are a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above), or R⁹ and R¹⁰ may be combined to form an oxo group, methylene group or a ring;
   X³ is a bond, oxygen atom, an optionally oxidized sulfur atom, N, NR^{7'} (wherein R^{7'} is a hydrogen, an optionally substituted hydrocarbon group, an optionally substituted hydroxyl group, an optionally substituted amino group, an optionally substituted heterocyclic group, or a group of the formula -Z^{1'}-Z² (wherein -Z^{1'}- is -CS-, -SO- or -SO₂-, and Z² is as defined above)), or an optionally substituted bivalent C₁₋₂ hydrocarbon group; and
   ----- is a single bond or a double bond;
   provided that at least one of R⁹ and R¹⁰ is CHR¹⁵R¹⁶ (wherein R¹⁵ and R¹⁶ are the same or different and are a hydrogen, an optionally substituted hydrocarbon group, an optionally substituted hydroxyl group, an optionally substituted amino group, an optionally substituted thiol group, a halogen atom, an optionally substituted heterocyclic group, or a group of the formula:-Z¹-Z² (wherein -Z¹- and Z² are as defined above)) and the other is other than an optionally substituted phenyl group; or a salt thereof or a prodrug thereof;
[5] The drug comprising the combination according to any one of the above-mentioned [1] to [4], wherein the bone resorption inhibitor is one or more medicines selected from the group consisting of (1) estrogen, (2) selective estrogen receptor modulators (SERM), (3) RANKL inhibitors, (4) strontium, (5) active vitamin D3, (6) vitamin K2, (7) ipriflavone preparations, (8) vitronectin receptor antagonists, (9) V-H+-ATPase inhibitors, (10) Src kinase inhibitors and (11) cathepsin K inhibitors;
[6] The drug comprising the combination according to any one of the above-mentioned [1] to [4], which is an agent for preventing or treating diseases caused by abnormality of calcium concentration or a calcium receptor in living body;
[7] The drug comprising the combination according to any one of the above-mentioned [1] to [4], which is an agent for preventing or treating bone diseases;
[8] The drug comprising the combination according to any one of the above-mentioned [1] to [4], which is an agent for preventing or treating osteoporosis or fracture;
[9] A method for preventing or treating diseases caused by abnormality of calcium concentration or a calcium receptor in living body which comprises administering to a mammal an effective amount of a calcium receptor modulators and an effective amount of a bone resorption inhibitor, wherein the calcium receptor modulator comprises a compound represented by the formula (I): 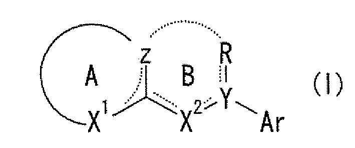 wherein ring A is an optionally substituted 5- to 7-membered ring;
   ring B is an optionally substituted 5- to 7-membered heterocyclic ring;
   X¹ is CR¹ (wherein R¹ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- is -CO-, -C-, - SO- or -SO₂-, and Z² is an optionally substituted hydrocarbon group, an optionally substituted heterocyclic group, an optionally substituted hydroxyl group, or an optionally substituted amino group)), CR¹R² (wherein R¹ is as defined above, R² is a hydrogen or an optionally substituted hydrocarbon group), N or NR¹³ (wherein R¹³ is a hydrogen, an optionally substituted hydrocarbon group, an optionally substituted hydroxyl group, an optionally substituted amino group, cyano group, a halogen atom, an optionally substituted heterocyclic group, or a group of the formula: -Z¹-Z² (wherein -Z¹- and Z² are as defined above));
   X² is N or NR³ (wherein R³ is a hydrogen, an optionally substituted hydrocarbon group, an optionally substituted hydroxyl group, an optionally substituted amino group, cyano group, a halogen atom, an optionally substituted heterocyclic group, or a group of the formula: -Z¹-Z² (wherein -Z¹- and Z² are as defined above);
   Y is C, CR⁴ (wherein R⁴ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above)) or N;
   Z is CR⁵ (wherein R⁵ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above)), CR⁵R⁶ (wherein, R⁵ is as defined above and R⁶ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above), and R⁵ and R⁶ may be the same or different), N or NR⁷ (wherein R⁷ is a hydrogen, an optionally substituted hydrocarbon group, an optionally substituted hydroxyl group, an optionally substituted amino group, cyano group, a halogen atom, an optionally substituted heterocyclic group, or a group of the formula: -Z¹-Z² (wherein -Z¹- and Z² are as defined above));
   Ar is an optionally substituted cyclic group;
   R is a hydrogen, an optionally substituted hydrocarbon group, an optionally substituted hydroxyl group, an optionally substituted amino group, an optionally substituted thiol group, an optionally substituted sulfonyl group or an optionally substituted sulfinyl group, or R and Z may be combined to form ring B; and
   ----- is a single bond or a double bond; or a salt thereof or a prodrug thereof; and
[10] Use of a calcium receptor modulator and a bone resorption inhibitor for manufacturing a drug for preventing or treating diseases caused by abnormality of calcium concentration or a calcium receptor in a living body, wherein the calcium receptor modulator is a compound represented by the formula (I): 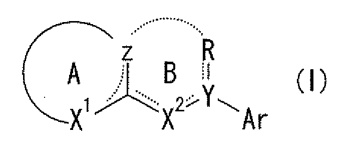 wherein ring A is an optionally substituted 5- to 7-membered ring;
   ring B is an optionally substituted 5- to 7-membered heterocyclic ring;
   X¹ is CR¹ (wherein R¹ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- is -CO-, -CS-, - SO- or -SO₂-, and Z² is an optionally substituted hydrocarbon group, an optionally substituted heterocyclic group, an optionally substituted hydroxyl group, or an optionally substituted amino group)), CR¹R² (wherein R¹ is as defined above, R² is a hydrogen or an optionally substituted hydrocarbon group), N or NR¹³ (wherein R¹³ is a hydrogen, an optionally substituted hydrocarbon group, an optionally substituted hydroxyl group, an optionally substituted amino group, cyano group, a halogen atom, an optionally substituted heterocyclic group, or a group of the formula: -Z¹-Z² (wherein -Z¹- and Z² are as defined above));
   X² is N car NR³ (wherein R³ is a hydrogen, an optionally substituted hydrocarbon group, an optionally substituted hydroxyl group, an optionally substituted amino group, cyano group, a halogen atom, an optionally substituted heterocyclic group, or a group of the formula: -Z¹-Z² (wherein -Z¹- and Z² are as defined above);
   Y is C, CR⁴ (wherein R⁴ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above)) or N;
   Z is CR⁵ (wherein R⁵ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above)), CR⁵R⁶ (wherein, R⁵ is as defined above and R⁶ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above), and R⁵ and R⁶ may be the same or different), N or NR⁷ (wherein R⁷ is a hydrogen, an optionally substituted hydrocarbon group, an optionally substituted hydroxyl group, an optionally substituted amino group, cyano group, a halogen atom, an optionally substituted heterocyclic group, or a group of the formula: -Z¹-Z² (wherein -Z¹- and Z² are as defined above));
   Ar is an optionally substituted cyclic group;
   R is a hydrogen, an optionally substituted hydrocarbon group, an optionally substituted hydroxyl group, an optionally substituted amino group, an optionally substituted thiol group, an optionally substituted sulfonyl group or an optionally substituted sulfinyl group, or R and Z may be combined to form ring B; and
   ----- is a single bond or a double bond; or a salt thereof or a prodrug thereof; and the like.

### Best Mode for Carrying Out the Invention

The above formula (I) includes a monocyclic heterocyclic compound containing ring A and a condensed heterocyclic compound containing rings A and B. In the above formulas, ring A of the formulas (I) and (II) is an optionally substituted 5- to 7-membered ring.

Examples of the "5- to 7-membered ring" of "an optionally substituted 5- to 7-membered ring" includes an aromatic or non-aromatic 5- to 7-membered hydrocarbon ring or 5- to 7-membered heterocyclic ring which may contain 1 to 3 heteroatoms selected from nitrogen, oxygen and sulfur atoms as the ring constituting atoms in addition to carbon atoms. Specific examples thereof include a hydrocarbon ring such as benzene, tropilidene, cyclopentane, cyclohexane, cycloheptane, 1-cyclopentene, 2-cyclopentene, 3-cyclopentene, 1-cyclohexene, 2-cyclohexene, 3-cyclohexene, 1-cycloheptene, 2-cycloheptene, 3-cycloheptene, 2,4-cycloheptadiene, etc.; a heterocyclic ring such as pyridine, pyrazine, pyrimidine, imidazole, furan, thiophene, dihydropyridine, diazepine, oxazepine, pyrrolidine, piperidine, hexamethylenimine, heptamethylenimine, tetrahydrofuran, piperazine, homopiperazine, tetrahydrooxazepine, morpholine, thiomorpholine, pyrrole, pyrazole, 1,2,3-triazole, oxazole, oxazolidine, thiazole, thiazolidine, isoxazole, imidazoline, triazole, thiadiazole, oxadiazole, oxathiadiazole, triazine, etc.; and the like.

Examples of the substituent(s) of "an optionally substituted 5- to 7-membered ring group" include halogen, nitro, cyano, oxo, an optionally substituted hydrocarbon group, an optionally substituted heterocyclic group, an optionally substituted sulfinyl group, an optionally substituted sulfonyl group, an optionally substituted hydroxyl group, an optionally substituted thiol group, an optionally substituted amino group, an optionally substituted acyl group, an optionally esterified or amidated carboxyl group, an optionally substituted phosphoryl group, or the like.

Examples of halogen include fluorine, chlorine, bromine, iodine, and the like, preferably, fluorine and chlorine.

Examples of the hydrocarbon group in an optionally substituted hydrocarbon group as the substituent of the 5-to 7-membered ring group include an optionally substituted aliphatic hydrocarbon group, an optionally substituted alicyclic hydrocarbon group, an optionally substituted alicyclic-aliphatic hydrocarbon group, an optionally substituted aromatic hydrocarbon group, an optionally substituted aromatic-aliphatic hydrocarbon group (an aralkyl group), and the like.

Examples of said aliphatic hydrocarbon group include a saturated aliphatic hydrocarbon group having 1-8 carbon atoms, (e.g., alkyl group) such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, etc.; and an unsaturated aliphatic hydrocarbon group having 2-8 carbon atoms (e.g., alkenyl group, alkynyl group, alkadienyl group, alkadiynyl group, etc.) such as vinyl, allyl, 1-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, 2,4-hexadienyl, 1-heptenyl, 1-octenyl, ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 2,4-hexadiynyl, 1-heptynyl, 1-octynyl, etc.

Examples of said alicyclic hydrocarbon group include a saturated alicyclic hydrocarbon group having 3-7 carbon atoms (e.g., cycloalkyl group, etc.) such as cyclopropyl, cyclobutyl, cyclopentyl, cyclohexyl, cycloheptyl, and the like; an unsaturated alicyclic hydrocarbon group having 3-7 carbon atoms (e.g., cycloalkenyl group, cycloalkadienyl group, etc.) such as 1-cyclopentenyl, 2-cyclopentenyl, 3-cyclopentenyl, 1-cyclohexenyl, 2-cyclohexenyl, 3-cyclohexenyl, 1-cycloheptenyl, 2-cycloheptenyl, 3-cycloheptenyl, 2,4-cycloheptadienyl, etc.; a partly saturated and fused bicyclic hydrocarbon group [preferably, C₉₋₁₀ partly saturated and fused bicyclic hydrocarbon group, etc. (including those where the benzene ring is combined to 5- or 6-membered non-aromatic cyclic hydrocarbon group)] such as 1-indenyl, 2-indenyl, 1-indanyl, 2-indanyl, 1,2,3,4-tetrahydro-1-naphthyl, 1,2,3,4-tetrahydro-2-naphthyl, 1,2-dihydro-1-naphthyl, 1,2-dihydro-2-naphthyl, 1,4-dihydro-1-naphthyl, 1,4-dihydro-2-naphthyl, 3,4-dihydro-1-naphthyl, 3,4-dihydro-2-naphthyl, etc.; and the like. Said alicyclic hydrocarbon group may be cross-linked.

Examples of said alicyclic-aliphatic hydrocarbon group include those where the above-mentioned alicyclic hydrocarbon group and the above-mentioned aliphatic hydrocarbon group are combined, for example, those having 4-14 carbon atoms such as cyclopropylmethyl, cyclopropylethyl, cyclobutylmethyl, cyclobutylethyl, cyclopentylmethyl, 2-cyclopentenylmethyl, 3-cyclopentenylmethyl, cyclopentylethyl, cyclohexylmethyl, 2-cyclohexenylmethyl, 3-cyclohexenylmethyl, cyclohexylethyl, cycloheptylmethyl, cycloheptylethyl, 2-(3,4-dihydro-2-naphtyl)ethyl, 2-(1,2,3,4-tetrahydro-2-naphtyl)ethyl, 2-(3,4-dihydro-2-naphtyl)ethenyl, etc. (e.g., C₃₋₇ cycloalkyl-C₁₋₄ alkyl group, C₃₋₇ cycloalkenyl-C₁₋₄ alkyl group, C₃₋₇ cycloalkyl-C₂₋₄ alkenyl group, C₃₋₇ cycloalkenyl-C₂₋₄ alkenyl group, C₉₋₁₀ partly saturated and fused bicyclic hydrocarbon-C₁₋₄ alkyl group, C₉₋₁₀ partly saturated and fused bicyclic hydrocarbon-C₂₋₄ alkenyl groups, etc.).

Examples of said aromatic hydrocarbon group include an aryl group having 6-10 carbon atoms (including that where a 5- to 6-membered non-aromatic hydrocarbon ring is fused with phenyl group) such as phenyl, α-naphthyl, β-naphthyl, 4-indenyl, 5-indenyl, 4-indanyl, 5-indanyl, 5,6,7,8-tetrahydro-1-naphthyl, 5,6,7,8-tetrahydro-2-naphthyl, 5,6-dihydro-1-naphthyl, 5,6-dihydro-2-naphthyl, 5,6-dihydro-3-naphthyl, 5,6-dihydro-4-naphthyl, etc.; and the like.

Examples of said aromatic-aliphatic hydrocarbon group include an aralkyl group having 7-14 carbon atoms (C₆₋₁₀ aryl-C₁₋₄ alkyl group) such as phenyl-C₁₋₄ alkyl group, e.g., benzyl, phenethyl, 1-phenylethyl, 1-phenylpropyl, 2-phenylpropyl, 3-phenylpropyl, etc.; naphthyl-C₁₋₄ alkyl group such as α-naphthylmethyl, α-naphthylethyl, β-naphthylmethyl, β-naphthylethyl, etc.; C₆₋₁₀ aryl-C₂₋₄ alkenyl group such as phenyl-C₂₋₄ alkenyl group, e.g., styryl, cinnamyl, etc.; and the like.

Examples of the heterocyclic group in an optionally substituted heterocyclic group as the substituent of the 5-to 7-membered ring include (i) a 5- to 7-membered heterocyclic group containing one sulfur atom, one nitrogen atom, or one oxygen atom, (ii) a 5- to 6-membered heterocyclic group containing 2-4 nitrogen atoms, (iii) a 5- to 6-membered heterocyclic group containing 1-2 nitrogen atoms and one sulfur or oxygen atom, or the like; and (iv) these heterocyclic groups may be fused with a 5- to 6-membered ring containing 2 or less nitrogen atoms, benzene ring, or a 5-membered ring containing one sulfur atom. In addition, each of the heterocyclic groups exemplified in (i) to (iv) may be a saturated or unsaturated heterocyclic group and the unsaturated heterocyclic group may be either aromatic or non-aromatic.

Examples of the heterocyclic group in an optionally substituted heterocyclic group as the substituent of the 5-to 7-membered ring include an aromatic monocyclic heterocyclic group, an aromatic fused heterocyclic group, and a non-aromatic heterocyclic group.

Specific examples of the heterocyclic group in an optionally substituted heterocyclic group as the substituent of the 5- to 7-membered ring include (i) an aromatic monocyclic heterocyclic group (e.g., furyl, thienyl, pyrrolyl, oxazolyl, isoxazolyl, thiazolyl, isothiazolyl, imidazolyl, pyrazolyl, 1,2,3-oxadiazalyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyrimidinyl, pyridazinyl, pyrazinyl, triazinyl, etc.); (ii) an aromatic fused heterocyclic group (e.g., benzofuranyl, isobenzofuranyl, benzo[b]thienyl, indolyl, isoindolyl, 1H-indazolyl, benzimidazolyl, benzoxazolyl, 1,2-benzisothiazolyl, 1H-benztriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxatinyl, thianthrenyl, phenanthredinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.); and (iii) a non-aromatic, heterocyclic group (e.g., oxiranyl, azetidinyl, oxetanyl, thietanyl, pyrrolidinyl, tetrahydrofuryl, thiolanyl, piperidyl, tetrahydropyranyl, morpholinyl, thiomorpholinyl, piperazinyl, etc.).

Examples of sulfinyl group in an optionally substituted sulfinyl group as the substituent of the 5- to 7-membered ring include that where -SO- is combined with "the hydrocarbon group" or "the heterocyclic group" in "an optionally substituted hydrocarbon group" or "an optionally substituted heterocyclic group" of the substituent of the 5- to 7-membered ring.

Preferred examples include a C₁₋₈ alkylsulfinyl group where sulfinyl group is combined with a C₁₋₈ alkyl group such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, etc.; a C₆₋₁₀ arylsulfinyl group where sulfinyl group is combined with a C₆₋₁₀ aryl group such as phenyl, α-naphthyl, β-naphthyl, 4-indenyl, 5-indenyl, 4-indanyl, 5-indanyl, 5,6,7,8-tetrahydro-1-naphthyl, 5,6,7,8-tetrahydro-2-naphthyl, 5,6-dihydro-1-naphthyl, 5,6-dihydro-2-naphthyl, 5,6-dihydro-3-naphthyl, 5,6-dihydro-4-naphthyl, etc.; a group where sulfinyl group is combined with an aromatic monocyclic heterocyclic group (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyrimidinyl, pyridazinyl, pyrazinyl, triazinyl, etc.); and a group where sulfinyl group is combined with an aromatic fused heterocyclic group (e.g., benzofuranyl, isobenzofuranyl, benzo[b]thienyl, indolyl, isoindolyl, 1H-indazolyl, benzimidazolyl, benzoxazolyl, 1,2-benzisothiazolyl, 1H-benztriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxatinyl, thianthrenyl, phenanthredinyl, phenanthrolinyl, indolizinyl, pyrrolo[1,2-b]pyridazinyl, pyrazolo[1,5-a]pyridyl, imidazo[1,2-a]pyridyl, imadazo[1,5-a]pyridyl, imidazo[1,2-b]pyridazinyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triaxolo[4,3-b]pyridazinyl, etc.). More preferred examples include a C₁₋₈ alkylsulfinyl group where sulfinyl group is combined with a C₁₋₈ alkyl group such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, etc.

Examples of sulfonyl group in an optionally substituted sulfonyl group as the substituent of the 5- to 7-membered ring include a group where -SO₂- is combined with "the hydrocarbon group" or "the heterocyclic group" in "an optionally substituted hydrocarbon group" or "an optionally substituted heterocyclic group" of the substituent of the 5- to 7-membered ring.

Preferred examples include a C₁₋₈ alkylsulfonyl group where sulfonyl group is combined with a C₁₋₈ alkyl group such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, etc.; a C₆₋₁₀ arylsulfonyl group where sulfonyl group is combined with a C₆₋₁₀ aryl group such as phenyl, α-naphthyl, β-naphthyl, 4-indenyl, 5-indenyl, 4-indanyl, 5-indanyl, 5,6,7,8-tetrahydro-1-naphthyl, 5,6,7,8-tetrahydro-2-naphthyl, 5,6-dihydro-1-naphthyl, 5,5-dihydro-2-naphthyl, 5,6-dihydro-3-naphthyl, 5,6-dihydro-4-naphthyl, etc.; a group where sulfonyl group is combined with an aromatic monocyclic heterocyclic group (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyrimidinyl, pyridazinyl, pyrazinyl, triazinyl, or the like); and a group where the sulfonyl group is combined with an aromatic, fused heterocyclic group (e.g., benzofuranyl, isobenzofuranyl, benzo[b]thienyl, indolyl, isoindolyl, 1H-indazolyl, benzimidazolyl, benzoxazolyl, 1,2-benzisothiazolyl, 1H-benztriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxatinyl, thianthrenyl, phenanthredinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.). More preferred examples include a C₁₋₈ alkylsulfonyl group where sulfonyl group is combined with a C₁₋₈ alkyl group such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, etc.

Examples of the optionally substituted hydroxyl group as the substituent of the 5- to 7-membered ring include hydroxyl group and that having an appropriate substituent, for example, "an optionally substituted hydrocarbon group" or "an optionally substituted heterocyclic group" of the above substituent of the 5- to 7-membered ring.

Preferred examples include a C₁₋₈ alkyloxy group whose substituent is a C₁₋₈ alkyl group such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, etc.; a C₆₋₁₀ aryloxy group whose substituent is a C₆₋₁₀ aryl group such as phenyl, α-naphthyl, β-naphthyl, 4-indenyl, 5-indenyl, 4-indanyl, 5-indanyl, 5,6,7,8-tetrahydro-1-naphthyl, 5,6,7,8-tetrahydro-2-naphthyl, 5,6-dihydro-1-naphthyl, 5,6-dihydro-2-naphthyl, 5,6-dihydro-3-naphthyl, 5,6-dihydro-4-naphthyl, etc.; a hydroxyl group substituted with an aromatic monocyclic heterocyclic group (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyrimidinyl, pyridazinyl, pyrazinyl, triazinyl, etc.); a hydroxyl group substituted with an aromatic fused heterocyclic group (e.g., benzofuranyl, isobenzofuranyl, benzo[b]thienyl, indolyl, isoindolyl, 1H-indazolyl, benzimidazolyl, benzoxazolyl, 1,2-benzisothiazolyl, 1H-benztriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxatinyl, thianthrenyl, phenanthredinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.).

More preferred examples include a C₆₋₁₀ aryloxy group (in particular, phenyloxy) or a hydroxyl group substituted with an aromatic monocyclic heterocyclic group (in particular, pyridyl) or an aromatic fused heterocyclic group (in particular, quinolyl).

"The hydrocarbon group" or "the heterocyclic group" as the substituent of the substituted hydroxyl group exemplified above may have the same substituent as that of "the hydrocarbon group" or "the heterocyclic group" in "an optionally substituted hydrocarbon group or an optionally substituted heterocyclic group" of the substituent of the 5- to 7-membered ring.

Examples of the optionally substituted thiol group as the substituent of the 5- to 7-membered ring include thiol group and that substituted with an appropriate group such as "an optionally substituted hydrocarbon group" or "an optionally substituted heterocyclic group" of the substituent of the 5- to 7-membered ring.

Preferred examples include a C₁₋₈ alkylthio group, whose substituent is a C₁₋₈ alkyl group such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, etc.; a C₆₋₁₀ arylthio group, whose substituent is a C₆₋₁₀ aryl group such as phenyl, α-naphthyl, β-naphthyl, 4-indenyl, 5-indenyl, 4-indanyl, 5-indanyl, 5,6,7,8-tetrahydro-1-naphthyl, 5,6,7,8-tetrahydro-2-naphthyl, 5,6-dihydro-1-naphthyl, 5,5-dihydro-2-naphthyl, 5,6-dihydro-3-naphthyl, 5,6-dihydro-4-naphthyl, etc.; a thiol group substituted with an aromatic monocyclic heterocyclic group (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyrimidinyl, pyridazinyl, pyrazinyl, triazinyl, etc.); and a thiol group substituted with an aromatic fused heterocyclic groups (e.g., benzofuranyl, isobenzofuranyl, benzo[b]thienyl, indolyl, isoindolyl, 1H-indazolyl, benzimidazolyl, benzoxazolyl, 1,2-benzisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxatinyl, thianthrenyl, phenanthredinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.).

"The hydrocarbon group" or "the heterocyclic group" as the substituent of the substituted thiol group exemplified above may have the same substituent as that of "the hydrocarbon group" or "the heterocyclic group" in "an optionally substituted hydrocarbon group" or "an optionally substituted heterocyclic group" of the substituent of the 5- to 7-membered ring.

More preferred examples include a C₁₋₈ alkylthio group substituted with a C₁₋₈ alkyl such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, or the like.

Examples of the optionally substituted amino group as the substituent of the 5- to 7-membered ring include amino group, an N-mono-substituted amino group, and an N,N-disubstituted amino group. Examples of said substituted amino groups include that having one or two substituents of an optionally substituted hydrocarbon group (e.g., the same group as an optionally substituted hydrocarbon group of the substituent of the 5- to 7-membered ring, more specifically, a C₁₋₈ alkyl group, a C₃₋₇ cycloalkyl group, a C₂₋₈ alkenyl group, a C₂₋₈ alkynyl group, a C₃₋₇ cycloalkenyl group, a C₆₋₁₀ aryl group that may have a C₁₋₄ alkyl group, etc.), an optionally substituted heterocyclic group (e.g., the same group as an optionally substituted heterocyclic group of the substituent of the 5- to 7-membered ring), or the formula: -COR' (wherein R' represents hydrogen atom or an optionally substituted hydrocarbon group or an optionally substituted heterocyclic group. As for "the hydrocarbon group" or "the heterocyclic group" in "an optionally substituted hydrocarbon group" or "an optionally substituted heterocyclic group" of R' may have the same substituent as that of "the hydrocarbon group" or "the heterocyclic group" in "an optionally substituted hydrocarbon group" or "an optionally substituted heterocyclic group" of the substituent of the 5- to 7-membered ring), preferably a C₁₋₁₀ acyl group (e.g., a C₂₋₇ alkanoyl, benzoyl, nicotinoyl, etc.). Specific examples thereof include methylamino, dimethylamino, ethylamino, diethylamino, dipropylamino, dibutylamino, diallylamino, cyclohexylamino, phenylamino, N-methyl-N-phenylamino, acetylamino, propionylamino, benzoylamino, nicotinoylamino, and the like.

In addition, the two groups in said substituted amino groups may be combined to form a nitrogen-containing 5- to 7-membered ring (e.g., piperidino, piperadino, morpholino, thiomorpholino, etc.).

Examples of the optionally substituted acyl group as the substituent of the 5- to 7-membered ring include (i) formyl or (ii) a group where the carbonyl group is combined with a C₁₋₁₀ alkyl group, a C₂₋₁₀ alkenyl group, a C₂₋₁₀ alkynyl group, a C₃₋₇ cycloalkyl group, a C₅₋₇ cycloalkenyl group, or an aromatic group (e.g., phenyl group, pyridyl group, etc.) (e.g., acetyl, propionyl, butyryl, isobytyryl, valeryl, isovaleryl, pivaloyl, hexanoyl, heptanoyl, octanoyl, cyclobutanecarbonyl, cyclopentanecarbonyl, cyclohexanecarbonyl, cycloheptanecarbonyl, crotonyl, 2-cyclohexenecarbonyl, benzoyl, etc.) and the like.

Examples of the optionally esterified carboxyl group as the substituent of the 5- to 7-membered ring include, in addition to carboxyl group, an alkyloxycarbonyl group, an alkenyloxycarbonyl, an alkynyloxycarbonyl, an aralkyloxycarbonyl group, an acyoxycarbonyl group, an aryloxycarbonyl group, and the like.

Examples of the alkyl group in said alkyloxycarbonyl group include a C₁₋₆ alkyl group (e.g., methyl, ethyl, propyl, isopropyl, butyl, isobutyl, sec-butyl, tert-butyl, etc.).

Examples of the alkenyl group in said alkenyloxycarbonyl group include a C₂₋₆ alkenyl group (e.g., vinyl, allyl, isopropenyl, 1-propenyl, 1-butenyl, 2-butenyl, 3-methylallyl, etc.).

Examples of the alkynyl group in said alkynyloxycarbonyl group include a C₂₋₆ alkynyl group (e.g., ethynyl, 2-propynyl, etc.).

The aralkyl group in said aralkyloxycarbonyl group means an aryl-alkyl group (e.g., C₆₋₁₀ aryl-C₁₋₆ alkyl, etc.). The aryl group in said aryl-alkyl group means a monocyclic or condensed polycyclic aromatic hydrocarbon group, and preferred examples include phenyl, naphthyl, anthryl, phenanthryl, acenaphthenyl, and the like. They may have a substituent such as a C₁₋₁₀ alkyl group, a C₂₋₁₀ alkenyl group, a C₂₋₁₀ alkynyl group, a C₃₋₈ cycloalkyl group, a C₃₋₈ cycloalkenyl group, a C₄₋₈ cycloalkadienyl group, an aryl group (e.g., C₆₋₁₄ aryl, etc.), an aromatic heterocyclic group (e.g., the same aromatic heterocyclic group as that of the substituent of the hydrocarbon group, the acyl group, the sulfonyl group, the sulfinyl group and the heterocyclic group of the above substituent of the 5- to 7-membered ring, etc.), a non-aromatic heterocyclic group (e.g., the same non-aromatic heterocyclic group as that of the substituent of the hydrocarbon group, the acyl group, the sulfinyl group, the sulfinyl group and the heterocyclic group of the above substituent of the 5- to 7-membered ring, etc.), an aralkyl group (e.g., a C₆₋₁₄ aryl-C₁₋₆ alkyl group, etc.), amino group, an N-mono-substituted amino group (e.g., the same N-mono-substituted amino group as that of the substituent of the hydrocarbon group, the acyl group, the sulfonyl group, the sulfinyl group and the heterocyclic group of the above substituent of the 5- to 7-membered ring, preferably a N-mono-C₁₋₄ alkylamino group, etc.), a N,N-disubstituted amino group (e.g., the same N,N-disubstituted amino group as that of the substituent in the hydrocarbon group, the acyl group, the sulfonyl group, the sulfinyl group and the heterocyclic group of the above substituent of the 5- to 7-membered ring, preferably a N,N-di-C₁₋₄ alkylamino group, etc.), amidino group, an acyl group (e.g., the same acyl group as that of the substituent of the hydrocarbon group, the acyl group, the sulfonyl group, the sulfinyl group and the heterocyclic group of the above substituent of the 5- to 7-membered ring, etc.), carbamoyl group, a N-mono-substituted carbamoyl group (e.g., a N-mono-C₁₋₄ alkyl-carbamoyl group such as methylcarbamoyl, ethylcarbamoyl, etc.; phenylcarbamoyl; etc.), a N,N-disubstituted carbamoyl group (a N,N-di-C₁₋₄ alkyl-carbamoyl group such as N,N-dimethylcarbamoyl, N,N-diethylcarbamoyl, etc.; piperidinocarbamoyl; morpholinocarbamoyl; etc.), sulfamoyl group, a N-mono-substituted sulfamoyl group (e.g., a N-mono-C₁₋₄ alkylsulfamoyl group such as methylsulfamoyl, ethylsulfamoyl, etc.; phenylsulfamoyl; p-toluenesulfamoyl; etc.), a N,N-disubstituted sulfamoyl group (e.g., a N,N-disubstituted C₁₋₄ alkylsulfamoyl group such as N,N-dimethylsulfamoyl, etc.; a N-C₁₋₄ alkyl-N-phenylsulfamoyl group such as N-methyl-N-phenylsulfamoyl, etc.; piperidinosulfamoyl; morpholinosulfamoyl; etc.), carboxyl group, a C₁₋₁₀ alkoxy-carbonyl group (e.g., methoxycarbonyl, ethoxycarbonyl, isopropoxycarbonyl, sec-butoxycarbonyl, isobutoxycarbonyl, tert-butoxycarbonyl, etc.), hydroxyl group, a C₁₋₁₀ alkoxy group, a C₂₋₁₀ alkenyloxy group, a C₃₋₇ cycloalkyloxy group, an aralkyloxy group (e.g., C₆₋₁₄ aryl-C₁₋₆ alkyloxy, etc.), an aryloxy group (e.g., C₆₋₁₄ aryloxy, etc.), mercapto group, a C₁₋₁₀ alkylthio group, an aralkylthio group (e.g., C₆₋₁₄ aryl-C₁₋₆ alkylthio, etc.), an arylthio group (e.g., C₆₋₁₄ arythio, etc.), sulfo group, cyano group, azido group, nitro group, nitroso group, a halogen atom, or the like. As for an alkyl group in said aryl-alkyl group, a C₁₋₆ alkyl group (e.g., methyl, ethyl, propyl, butyl, etc.) is preferred. Preferred examples of said aralkyl group, i.e., an aryl-alkyl group include benzyl, phenethyl, 3-phenylpropyl, (1-naphthyl)methyl, (2-naphthyl)methyl, and the like. Among them, benzyl, phenethyl, and the like are preferred.

As the acyl group in said acyloxycarbonyl group, for example, formyl, a C₂₋₄ alkanoyl group, a C₃₋₄ alkenoyl group, a C₃₋₄ alkynoyl group and the like are exemplified.

As the aryl group in said aryloxycarbonyl group, phenyl, naphthyl and the like are exemplified.

Examples of the optionally amidated carboxyl group as the substituent for the hydrocarbon group, acyl group, sulfonyl group, sulfinyl group and heterocyclic group that are the substituents of the 5- to 7-membered ring include a carboxyl group amidated with an optionally substituted amino group as the substituent for the optionally substituted hydrocarbon group, acyl group, sulfonyl group and heterocyclic group that are the substituents of the above 5- to 7-membered ring.

Example of the optionally substituted phosphoryl group of the substituent of the 5- to 7-membered ring include phosphoryl group, a (C₁₋₆ alkoxy)phosphoryl group such as ethoxyphosphoryl, a di-(C₁₋₆ alkoxy)phosphoryl group such as diethoxyphosphoryl, etc.; a lower (C₁₋₆) alkyl group substituted with an optionally esterified phosphono group such as a phosphono-C₁₋₆ alkyl group, a C₁₋₆ alkoxyphosphoryl-C₁₋₆ alkyl group, a di- (C₁₋₆ alkoxy) phosphoryl-C₁₋₆ alkyl group such as diethoxyphosphorylmethyl, etc.; and the like.

"The hydrocarbon group", "the heterocyclic group", "the sulfinyl group", or "the sulfonyl group" in "an optionally substituted hydrocarbon group", "an optionally substituted heterocyclic group", "an optionally substituted sulfinyl group", or "an optionally substituted sulfonyl group" of the substituent of the 5- to 7-membered ring may be further substituted with 1 to 3 substituents. Examples of said substituents include a lower (C₁₋₆) alkyl group (e.g., methyl, ethyl, propyl, isopropyl, butyl, isobutyl, sec-butyl., tert-butyl, pentyl, isopentyl, neopentyl, hexyl, etc.); a lower (C₂₋₆) alkenyl group (e.g., vinyl, allyl, 1-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 4-hexenyl, 5-hexenyl, etc.); a lower (C₂₋₆) alkynyl group (e.g., ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, etc.); a C₃₋₇ cycloalkyl group (e.g., cyclopropyl, cyclobutyl, cyclopentyl, cyclohexyl, cycloheptyl, etc.); a C₆₋₁₀ aryl group (e.g., phenyl, α-naphthyl, β-naphthyl, etc.); an aromatic heterocyclic group [e.g., (i) an aromatic 5- or 6-membered heterocyclic group having 1-4 heteroatoms selected from nitrogen atom, oxygen atom, and sulfur atom; (ii) a fused bicyclic heterocyclic group formed by condensation of an aromatic 5- or 6-membered heterocyclic group having 1 to 3 heteroatoms selected from nitrogen atom, oxygen atom, and sulfur atom with benzene ring or an aromatic 5- or 6-membered heterocyclic group having 1 to 3 heteroatoms selected from nitrogen atom, oxygen atom, and sulfur atom; (iii) a fused tricyclic heterocyclic group formed by condensation of [1] an aromatic, 5- or 6-membered heterocyclic group having 1-3 heteroatoms selected from nitrogen atom, oxygen atom and sulfur atom, [2] benzene ring, and [3] an aromatic 5- or 6-membered heterocyclic group having 1-3 heteroatoms selected from nitrogen atom, oxygen atom and sulfur atom or benzene ring, such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyrimidinyl, pyridazinyl, pyrazinyl, triazinyl, benzofuranyl, isobenzofuranyl, benzo[b]thienyl, indolyl, isoindolyl, 1H-indazolyl, benzimidazolyl, benzoxazolyl, 1,2-benzisothiazolyl, 1H-benztriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxatinyl, thianthrenyl, phenanthredinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.]; a heterocyclic-oxy group formed by combining each of the above heterocyclic groups (i), (ii) and (iii) with oxy group; a non-aromatic heterocyclic group (e.g., a non-aromatic, 4- or 7-membered heterocyclic group having 1 to 3 heteroatoms selected from nitrogen atom, oxygen atom and sulfur atom, such as oxiranyl, azetidinyl, oxetanyl, thietanyl, pyrrolidinyl, tetrahydrofuryl, thiolanyl, piperidinyl, tetrahydropyranyl, morpholinyl, thiomorpholinyl, piperazinyl, etc.); a C₇₋₁₄ aralkyl group (e.g., a C₆₋₁₀ aryl-C₁₋₄ alkyl group such as benzyl, phenethyl, 1-phenylethyl, 1-phenylpropyl, 2-phenylpropyl, 3-phenylpropyl, α-naphthylmethyl, α-naphthylethyl, β-naphthylmethyl, β-naphthylethyl, etc.); amino group; a N-mono-substituted amino group [e.g., a N-(C₁₋₆ alkyl)amino group such as methylamino, ethylamino, allylamino, cyclohexylamino, phenylamino, a N- (C₂₋₆ alkenyl)amino group, a N-(C₃₋₇ cycloalkyl)amino group, a N-(C₆₋₁₀ aryl)amino group, etc.]; a N,N-disubstituted amino group [e.g., an amino group substituted with two substituents selected from a C₁₋₆ alkyl group, a C₂₋₆ alkenyl group, a C₃₋₇ cycloalkenyl group, and a C₆₋₁₀ aryl group, such as dimethylamino, diethylamino, dibutylamino, diallylamino, N-methyl-N-phenylamino, etc.]; amidino group; an acyl group (e.g., formyl, a C₂₋₈ alkanoyl group such as acetyl, propionyl, butyryl, isobytyryl, valeryl, isovaleryl, pivaloyl, hexanoyl, heptanoyl, octanoyl, cyclopropanecarbonyl, cyclobutanecarbonyl, cyclopentanecarbonyl, cyclohexanecarbonyl, crotonyl, 2-cyclohexenecarbonyl, benzoyl, nicotinoyl, etc.; a C₃₋₈ alkenoyl group; a C₃₋₇ cycloalkyl-carbonyl group; a C₃₋₇ cycloalkenyl-carbonyl group; a C₆₋₁₀ aryl-carbonyl group; a heterocyclic-carbonyl group formed by binding of an aromatic or non-aromatic 5- or 6-membered heterocyclic group having 1-3 heteroatoms selected from nitrogen atom, oxygen atom and sulfur atom with carbonyl group, etc.); carbamoyl group; a mono-substituted carbamoyl group [e.g., a N-(C₁₋₆ alkyl)carbamoyl group such as methylcarbamoyl, ethylcarbamoyl, cyclohexylcarbamoyl, phenylcarbamoyl, etc.]; a N- (C₂₋₆ alkenyl)carbamoyl group; a N- (C₃₋₇ cycloalkyl)carbamoyl group; a N-(C₆₋₁₀ aryl) carbamoyl group; etc.]; a N,N-disubstituted carbamoyl group [e.g., a carbamoyl group substituted with two substituents selected from a C₁₋₆ alkyl group, a C₂₋₆ alkenyl group, a C₃₋₇ cycloalkyl group, and a C₆₋₁₀ aryl group, such as dimethylcarbamoyl, diethylcarbamoyl, dibutylcarbamoyl, diallylcarbamoyl, N-methyl-N-phenylcarbamoyl, etc.]; sulfamoyl group, a N-mono-substituted sulfamoyl group [e.g., a N-(C₁₋₆ alkyl)sulfamoyl group such as methylsulfamoyl, ethylsulfamoyl, cyclohexylsulfamoyl, phenylsulfamoyl, etc.; a N-(C₂₋₆ alkenyl) sulfamoyl group; a N-(C₃₋₇ cycloalkyl)sulfamoyl group; a N-(C₆₋₁₀ aryl) sulfamoyl group; etc.], a N,N-disubstituted sulfamoyl group [e.g., sulfamoyl group substituted with two substituents selected from a C₁₋₆ alkyl group, a C₂₋₆ alkenyl group, a C₃₋₇ cycloalkyl group, and a C₆₋₁₀ aryl group, such as dimethylsulfamoyl, diethylsulfamoyl, dibutylsulfamoyl, diallylsulfamoyl, N-methyl-N-phenylsulfamoyl, etc.]; carboxyl group; a lower (C₁₋₆) alkoxy-carbonyl group (e.g., methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl, etc.); hydroxyl group; a lower (C₁₋₆) alkoxy group (e.g., methoxy, ethoxy, propoxy, isopropoxy, butoxy, isobutoxy, sec-butoxy, tert-butoxy, pentyloxy, hexyloxy, etc.); a lower (C₂₋₁₀) alkenyloxy group (e.g., allyloxy, 2-butenyloxy, 2-pentenyloxy, 3-hexenyloxy, etc.); a C₃₋₇ cycloalkyloxy group (e.g., cyclopropyloxy, cyclobutyloxy, cyclopentyloxy, cyclohexyloxy, cycloheptyloxy, etc.); a C₆₋₁₀ aryloxy group (e.g., phenoxy, naphthyloxy, etc.); a C₇₋₁₄ aralkyloxy group (e.g., a C₆₋₁₀ aryl-C₁₋₄ alkyloxy group such as phenyl-C₁₋₄ alkyloxy, naphthyl-C₁₋₄ alkyloxy, etc.); mercapto group; a lower (C₁₋₆)alkylthio group (e.g., methylthio, ethylthio, propylthio, isopropylthio, butylthio, isobutylthio, *sec-*butylthio, tert-butylthio, pentylthio, isopentylthio, neopentylthio, hexylthio, etc.), a C₇₋₁₄ aralkylthio group (e.g., a C₆₋₁₀ aryl-C₁₋₄ alkylthio group such as phenyl-C₁₋₄ alkylthio, naphthyl-C₁₋₄ alkylthio, etc.); a C₆₋₁₀ arylthio group (e.g., phenylthio, naphtylthio, etc.), a lower (C₁₋₆) alkylsulfinyl group (e.g., methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, isopentylsulfinyl, neopentylsulfinyl, hexylsulfinyl, etc.); a C₇₋₁₄ aralkylsulfinyl group (e.g., a C₆₋₁₀ aryl-C₁₋₄ alkylsulfinyl group such as phenyl-C₁₋₄ alkylsulfinyl, naphthyl-C₁₋₄ alkylsulfinyl, etc.); a C₆₋₁₀ arylsulfinyl group (e.g., phenylsulfinyl, naphtylsulfinyl, etc.); a lower (C₁₋₆) alkylsulfonyl group (e.g., methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, isopentylsulfonyl, neopentylsulfonyl, hexylsulfonyl, etc.), a C₇₋₁₄ aralkylsulfonyl group (e.g., a C₆₋₁₀ aryl-C₁₋₄ alkylsulfonyl group such as phenyl-C₁₋₄ alkylsulfonyl, naphthyl-C₁₋₄ alkylsulfonyl, etc.), a C₆₋₁₀ arylsulfonyl group (e.g., phenylsulfonyl, naphtylsulfonyl, etc.); sulfo group; cyano group; azido group; a halogen atom (e.g., fluorine, chlorine, bromine, iodine, etc.); nitro group; nitroso group; an optionally esterified phosphono group [e.g., phosphono group, a (C₁₋₆ alkoxy)phosphoryl group such as ethoxyphosphoryl, a di-(C₁₋₆ alkoxy)phosphoryl group such as diethoxyphosphoryl, etc.]; a lower (C₁₋₆) alkyl group substituted with an optionally esterified phosphono group (e.g., a phosphono-C₁₋₆ alkyl group, a C₁₋₆ alkoxyphosphoryl-C₁₋₆ alkyl group, a di-(C₁₋₆ alkoxy)phosphoryl-C₁₋₆ alkyl group such as diethoxyphosphorylmethyl, etc.); a C₁₋₆ haloalkyl group (e.g., a C₁₋₆ alkyl group substituted with 1 to 4 halogen such as trifluoromethyl, etc.); a C₁₋₆ haloalkoxy group (e.g., a C₁₋₆ alkoxy group substituted with 1 to 4 halogen such as trifluoromethoxy, etc.); and the like.

Among the above substituents, when hydroxyl group is located adjacent to a lower (C₁₋₆) alkoxy group, they may form C₁₋₆ alkylenedioxy groups such as methylenedioxy, ethylenedioxy, or the like.

The above C₆₋₁₀ aryl group, the C₆₋₁₀ aryl group as a substituent of the aromatic heterocyclic group and the N-mono-substituted amino group, the C₆₋₁₀ aryl group as a substituent of the N,N-di-substituted amino group, the C₆₋₁₀ aryl group as a substituent of the N-mono-substituted carbamoyl group, the C₆₋₁₀ aryl group as a substituent of the N,N-di-substituted carbamoyl group, the C₆₋₁₀ aryl as a substituent of the N-mono-substituted sulfamoyl group, the C₆₋₁₀ aryl group as a substituent of the N,N-disubstituted sulfamoyl group, the C₆₋₁₀ aryl group as a substituent of the C₆₋₁₀ aryloxy group, the C₆₋₁₀ aryl group of the C₇₋₁₄ aralkyloxy group, the C₆₋₁₀ aryl group of the C₇₋₁₄ aralkylthio groups, the C₆₋₁₀ aryl group of the C₆₋₁₀ arylthio groups, the C₆₋₁₀ aryl group of the C₇₋₁₄ aralkylsulfinyl groups, the C₆₋₁₀ aryl group of the C₆₋₁₀ arylsulfinyl group, the C₆₋₁₀ aryl group of the C₇₋₁₄ aralkylsulfonyl groups, and the C₆₋₁₀ aryl group in the C₆₋₁₀ arylsulfonyl group may be substituted further with 1 to 3 substituents. Examples of said substituent include a lower (C₁₋₆) alkyl group, amino group, a N-(C₁₋₆ alkyl)amino group, a N,N-di-(C₁₋₆ alkyl)amino group, amidino group, carbamoyl group, a N-(C₁₋₆ alkyl) carbamoyl group, a N,N-di-(C₁₋₆ alkyl)carbamoyl group, sulfamoyl group, a N-(C₁₋₆ alkyl)sulfamoyl group, a N,N-di-(C₁₋₆ alkyl)sulfamoyl group, carboxyl group, a lower (C₂₋₇) alkoxycarbonyl group, hydroxyl group, a lower (C₁₋₆) alkoxy group, mercapto group, a lower (C₁₋₆) alkylthio group, sulfo group, cyano group, azido group, a halogen atom, nitro group, nitroso group, an optionally substituted phosphono group [e.g., phosphono group, a C₁₋₆ alkoxyphosphoryl group, a di-(C₁₋₆ alkoxy)phosphoryl group, etc.], a lower (C₁₋₆) alkyl group substituted with an optionally esterified phosphono group [e.g., a phosphono-C₁₋₆ alkyl group, a C₁₋₆ alkoxyphosphoryl-C₁₋₆ alkyl group, a di-(C₁₋₆ alkoxy)phosphoryl-C₁₋₆ alkyl group such as diethoxyphosphorylmethyl, etc.], and the like.

Among the above substituent, when hydroxyl group is located adjacent to a lower (C₁₋₆) alkoxyl group, they may form a C₁₋₆ alkylenedioxy group such as methylenedioxy, ethylenedioxy, or the like.

The number of the substituents of the 5- to 7-membered ring is 1 to 3, preferably 1 to 2 and the substituents may be the same or different and present at any possible positions of the ring.

Q of the formula (II) is C, CR⁵, or N.

R⁵ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- is -CO-, -CS-, -SO- or - SO₂-, and Z² is an optionally substituted hydrocarbon group, an optionally substituted heterocyclic group, an optionally substituted hydroxyl group, or an optionally substituted amino group).

Examples of the optionally substituted hydrocarbon group, the optionally substituted hydroxyl group, the optionally substituted amino group and the optionally substituted heterocyclic group of R⁵ or Z² include the same groups as those exemplified with respect to the above substituents of the 5- to 7-membered ring in ring A.

Examples of halogen and the optionally substituted thiol group of R⁵ include the same groups as those exemplified with respect to the above substituent of the 5-to 7-membered ring in ring A. X¹ in the formulas (I) and (II) is CR¹, CR¹R², N or NR¹³.

R¹ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above).

Examples of the optionally substituted hydrocarbon group, the optionally substituted hydroxyl group, the optionally substituted amino group, the optionally substituted thiol group, halogen and the optionally substituted heterocyclic group of R¹ include the same groups as those exemplified with respect to the substituents of the 5- to 7-membered ring in ring A.

R² is a hydrogen, or an optionally substituted hydrocarbon group, and examples of the optionally substituted hydrocarbon group of R² include the same group as that exemplified with respect to the substituent of the 5- to 7-membered ring in ring A.

R¹³ is a hydrogen, an optionally substituted hydrocarbon group, an optionally substituted hydroxyl group, an optionally substituted amino group, an optionally substituted heterocyclic group, or a group of the formula: -Z¹-Z² (wherein -Z¹- and Z² are as defined above) .

Examples of the optionally substituted hydrocarbon group, the optionally substituted hydroxyl group, the optionally substituted amino group and the optionally substituted heterocyclic group of R¹³ include the same groups as those exemplified with respect to the substituents of the 5- to 7-membered ring in ring A.

R¹ of the formula (III) is a hydrogen, an optionally substituted hydrocarbon group, an optionally substituted hydroxyl group, an optionally substituted thiol group, an optionally substituted amino group, cyano group, a halogen atom, an optionally substituted heterocyclic group, or a group of the formula: -Z¹-Z² (wherein -Z¹- is -CO-, -CS-, - SO- or -SO₂-, and Z² is an optionally substituted hydrocarbon group, an optionally substituted heterocyclic group, an optionally substituted hydroxyl group, or an optionally substituted amino group).

In R¹ of the formula (III), preferred example of the group of the formula: -Z¹-Z² is a group of the formula: - CONR²⁰(CR²¹R²²R²³), wherein R²⁰ is a hydrogen or an optionally substituted hydrocarbon group, and R²¹, R²², and R²³ are the same or different and are an optionally substituted hydrocarbon group, an optionally substituted heterocyclic group, or R²⁰ and R²¹ may be combined to form a ring.

Examples of the optionally substituted hydrocarbon group of R¹, R²⁰, R²¹, R²² and R²³, the optionally substituted heterocyclic group of R¹, R²¹, R²² and R²³, and the optionally substituted hydroxyl group, the optionally substituted amino group, the optionally substituted thiol group and halogen of R¹ include the same groups as those exemplified with respect to the substituents of the 5- to 7-membered ring in ring A of the formulas (I) and (II).

Preferably, at least one of R²¹, R²² and R²³ is an optionally substituted heterocyclic group which may be fused with an optionally substituted benzene ring, or an optionally substituted phenyl group which may be fused with an optionally substituted aromatic heterocyclic ring.

Examples of the "fused heterocyclic group" of the "optionally substituted heterocyclic group which may be fused with an optionally substituted benzene ring" and the "fused phenyl group" of the " optionally substituted phenyl group which may be fused with an optionally substituted aromatic heterocyclic ring" of R²¹, R²² and R²³ include the same groups as those exemplified with respect to the aromatic fused heterocyclic group as the substituents of the 5- to 7-membered ring in ring A.

Examples of these substituents include the same groups as those exemplified with respect to the substituents of the 5- to 7-membered ring in ring A of the formulas (I) and (II).

The ring formed in combination with R²⁰ and R²¹ is preferably an optionally substituted 5- to 7-membered ring, more preferably an optionally substituted 5- or 6-membered ring, and may be fused with an optionally substituted benzene ring. Such rings include the same rings as those exemplified with respect to the "5- to 7-membered ring" of "an optionally substituted 5- to 7-membered ring" in the ring A of the formulas (I) and (II).

These rings may have 1 to 3 substituents selected from the group consisting of (1) halogen, (2) hydrogen, (3) a phenoxy which may be substituted with 1 to 3 substituents selected from halogen, hydroxyl, C₁₋₆ alkyl, C₁₋₆ alkoxy, C₁₋₆ acyl (e.g., formyl, C₂₋₆ alkanoyl, etc.), cyano, amino, mono-C₁₋₆ alkyl-amino, di-C₁₋₆ alkyl-amino, C₁₋₆ alkyl-sulfanyl, C₁₋₆ alkyl-sulfinyl, C₁₋₆ alkyl-sulfonyl, C₁₋₆ alkoxy-carbonyl, carbamoyl, N-C₁₋₆ alkyl-carbamoyl and N,N-di-C₁₋₆ alkyl-carbamoyl,
(4) C₁₋₆ alkoxy which may be substituted with 1 to 3 substituents selected from halogen, hydroxyl, C₁₋₆ alkyl, C₁₋₆ alkoxy, C₁₋₆ acyl, cyano, amino, mono-C₁₋₆ alkyl-amino, di-C₁₋₆ alkyl-amino, C₁₋₆ alkyl-sulfanyl, C₁₋₆ alkyl-sulfinyl, C₁₋₆ alkyl-sulfonyl, C₁₋₆ alkoxy-carbonyl, carbamoyl, N-C₁₋₆ alkyl-carbamoyl, N,N-di-C₁₋₆ alkyl-carbamoyl and phenyl which may be substituted with 1 to 3 substituents selected from halogen, hydroxyl, C₁₋₆ alkyl, C₁₋₆ alkoxy, C₁₋₆ acyl, cyano, halogeno C₁₋₆ alkyl (e.g., trifluoromethyl, etc.), amino, mono-C₁₋₆ alkyl-amino, di-C₁₋₆ alkyl-amino, C₁₋₆ alkyl-sulfanyl, C₁₋₆ alkyl-sulfinyl, C₁₋₆ alkyl-sulfonyl, C₁₋₆ alkoxy-carbonyl, carbamoyl, N-C₁₋₆ alkyl-carbamoyl and N,N-di-C₁₋₆ alkyl-carbamoyl,
and (5) a C₁₋₈ hydrocarbon group (e.g., C₁₋₈ alkyl, C₃₋₇ cycloalkyl, C₂₋₈ alkenyl, C₂₋₈ alkynyl, C₃₋₇ cycloalkenyl, etc.) which may be substituted with 1 to 3 substituents selected from halogen, hydroxyl, C₁₋₆ alkyl, C₁₋₆ alkoxy, C₁₋₆ acyl, cyano, amino, mono-C₁₋₆ alkyl-amino, di-C₁₋₆ alkyl-amino, C₁₋₆ alkyl-sulfanyl, C₁₋₆ alkyl-sulfinyl, C₁₋₆ alkyl-sulfonyl, C₁₋₆ alkoxy-carbonyl, carbamoyl, N-C₁₋₆ alkyl-carbamoyl, N,N-di-C₁₋₆, alkyl-carbamoyl and phenyl which may be substituted with 1 to 3 sunstitutents selected from halogen, hydroxyl, C₁₋₆ alkyl, C₁₋₆ alkoxy, C₁₋₆ acyl, cyano, halogeno C₁₋₆ alkyl (e.g., trifluoromethyl, etc.), amino, mono-C₁₋₆ alkyl-amino, di-C₁₋₆ alkyl-amino, C₁₋₆ alkyl-sulfanyl, C₁₋₆ alkyl-sulfinyl, C₁₋₆ alkyl-sulfonyl, C₁₋₆ alkoxy-carbonyl, carbamoyl, N-C₁₋₆ alkyl-carbamoyl and N,N-di-C₁₋₆ alkyl-carbamoyl.

Specific examples of these substituents include the same groups as those exemplified with respect to the substituents of the 5- to 7-membered ring in ring A of the formulas (I) and (II).

R^{1a} of the formula (IIIa) is (1) an optionally substituted heterocyclic group or (2) a group of the formula: -Z^{1a}-Z^{2a} (wherein -Z^{1a}- is -CO-, -CS-, -SO- or -SO₂-, and Z^{2a} is (i) an optionally substituted heterocyclic group, (ii) -NR^{20a}(CR^{21a}R^{22a}R^{23a}) (wherein (a) R^{20a} is a hydrogen or an optionally substituted hydrocarbon group; and R^{21a} is an optionally substituted heterocyclic group which may be fused with an optionally substituted benzene ring, or an optionally substituted C₆₋₁₀ aryl group which may be fused with an optionally substituted aromatic heterocyclic ring and R^{22a} and R^{23a} are the same or different and are an optionally substituted hydrocarbon group or an optionally substituted heterocyclic group or R^{22a} and R^{23a} may be combined to form a ring, or (b) R^{20a} is a hydrogen or an optionally substituted hydrocarbon group; and R^{21a}, R^{22a} and R^{23a} are the same or different and are an optionally substituted C₁₋₈ aliphatic hydrocarbon group, provided that the sum total of the number of carbon atoms is 7 or more), (iii) -NR^{20a}R^{25a} (wherein R^{20a} is as defined above and R^{25a} is an optionally substituted C₆₋₁₀ aryl-C₂₋₄ alkyl, C₆₋₁₀ aryloxy-C₂₋₄ alkyl, C₆₋₁₀ arylamino-C₂₋₄ alkyl, C₇₋₁₄ aralkylamino-C₂₋₄ alkyl, heterocyclic ring-C₂₋₄ alkyl or heterocyclic group), (iv) a substituted 5- to 7-membered cyclic amino group, or (v) -OR^{24a} (wherein R^{24a} is (a) an optionally substituted C₇₋₁₄ aralkyl group, (b) an optionally substituted C₃₋₇ alicyclic hydrocarbon group, (c) an optionally substituted C₇₋₂₄ aliphatic hydrocarbon group, or (d) an optionally substituted heterocyclic group)).

Examples of the optionally substituted hydrocarbon group of R^{20a}, R^{22a} and R^{23a} and the optionally substituted heterocyclic group of R^{1a}, Z^{2a}, R^{21a}, R^{22a} and R^{23a} include the same groups as those exemplified with respect to the substituents of the 5- to 7-membered ring in ring A of the formulas (I) and (II).

Examples of the "fused heterocyclic group" of the "optionally substituted heterocyclic group which may be fused with an optionally substituted benzene ring" and the "fused phenyl group" of the "optionally substituted phenyl group which may be fused with an optionally substituted aromatic heterocyclic ring" of R^{21a}, R^{22a} and R^{22a} include the same groups as those exemplified with respect to the aromatic fused heterocyclic group as the substituents of the 5- to 7-membered ring in ring A.

Examples of the ring formed in combination with R^{20a} and R^{21a} and the substituents thereof include the same rings and substituents as those exemplified with respect to the ring formed in combination with R²⁰ and R²¹ and the substituents thereof.

Examples of the "optionally substituted C₁₋₈ aliphatic hydrocarbon group" of R^{20a} include the same groups as those exemplified with respect to the aliphatic hydrocarbon group as the substituents of the 5- to 7-membered ring in ring A.

Examples of the "optionally substituted C₇₋₁₄ aralkyl group", the "optionally substituted C₃₋₇ alicyclic hydrocarbon group" and the "optionally substituted heterocyclic group" of R^{24a} include the same groups as those exemplified with respect to the substituents of the 5- to 7-membered ring in ring A, respectively.

Examples of the "C₇₋₂₄ aliphatic hydrocarbon group" of the "optionally substituted C₇₋₂₄ aliphatic hydrocarbon group" in R^{24a} include, for example, C₇₋₂₄ alkyl, C₇₋₂₄ alkenyl, C₇₋₂₄ alkynyl, C₇₋₂₄ alkadienyl, C₇₋₂₄ alkadiynyl such as heptyl, octyl, 1-heptenyl, 1-octenyl, 1-heptynyl, 1-octynyl, etc.

Examples of the substituents of the "optionally substituted C₇₋₂₄ aliphatic hydrocarbon group" in R^{24a} include the same substituents as those exemplified with respect to the substituents of the hydrocarbon group as the substituents of 5- to 7-membered ring in ring A.

In the formula (IIIa), R^{1a} is preferably (1) an optionally substituted 5- to 7-membered aromatic or non-aromatic heterocyclic group having 1-4 hetero atoms selected from nitrogen atom, oxygen atom and sulfur atom, or (2) a group of the formula: -CO-Z^{2c} (wherein Z^{2c} is (i) an optionally substituted 5- to 7-membered aromatic or non-aromatic heterocyclic group having 1-4 hetero atoms selected from nitrogen atom, oxygen atom and sulfur atom, (ii) -NR²⁰C(CR²¹CR^{22c}R^{23c}) (wherein (a) R^{20c} is a hydrogen or an optionally substituted hydrocarbon group selected from C₁₋₈ saturated aliphatic hydrocarbon group, C₂₋₈ unsaturated aliphatic hydrocarbon group, C₃₋₇ saturated alicyclic hydrocarbon group, C₃₋₇ unsaturated alicyclic hydrocarbon group, C₉₋₁₀ partly saturated and fused bicyclic hydrocarbon group, C₃₋₇ saturated or unsaturated alicyclic-C₁₋₈ saturated or unsaturated aliphatic hydrocarbon group, C₉₋₁₀ partly saturated and fused bicyclic hydrocarbon-C₁₋₄ alkyl group, C₉₋₁₀ partly saturated and fused bicyclic hydrocarbon-C₂₋₄ alkenyl group, C₆₋₁₀ aryl group and C₇₋₁₄ aralkyl group; and R^{21c} is 1) an optionally substituted 5- to 7-membered aromatic or non-aromatic heterocyclic group having 1-4 hetero atoms selected from nitrogen atom, oxygen atom and sulfur atom, which may be fused with an optionally substituted benzene ring, or 2) an optionally substituted C₆₋₁₀ aryl group (e.g., phenyl group, etc.) which may be fused with an optionally substituted 5- to 7-membered aromatic heterocyclic ring having 1-4 hetero atoms selected from nitrogen atom, oxygen atom and sulfur atom; and R^{22c} and R^{23c} are the same or different and are an optionally substituted hydrocarbon group selected from C₁₋₈ saturated aliphatic hydrocarbon group, C₂₋₈ unsaturated aliphatic hydrocarbon group, C₃₋₇ saturated alicyclic hydrocarbon group, C₃₋₇ unsaturated alicyclic hydrocarbon group, C₉₋₁₀ partly saturated and fused bicyclic hydrocarbon group, C₃₋₇ saturated or unsaturated alicyclic-C₁₋₈ saturated or unsaturated aliphatic hydrocarbon group, C₉₋₁₀ partly saturated and fused bicyclic hydrocarbon-C₁₋₄ alkyl group, C₉₋₁₀ partly saturated and fused bicyclic hydrocarbon-C₂₋₄ alkenyl group, C₆₋₁₀ aryl group and C₇₋₁₄ aralkyl group, or an optionally substituted 5- to 7-membered aromatic or non-aromatic heterocyclic group having 1-4 hetero atoms selected from nitrogen atom, oxygen atom and sulfur atom or R^{22c} and R^{23c} may be combined to form a C₃₋₇ carbon ring, or (b) R^{20c} is a hydrogen or an optionally substituted hydrocarbon group selected from C₁₋₈ saturated aliphatic hydrocarbon group, C₂₋₈ unsaturated aliphatic hydrocarbon group, C₃₋₇ saturated alicyclic hydrocarbon group, C₃₋₇ unsaturated alicyclic hydrocarbon group, C₉₋₁₀ partly saturated and fused bicyclic hydrocarbon group, C₃₋₇ saturated or unsaturated alicyclic-C₁₋₈ saturated or unsaturated aliphatic hydrocarbon group, C₉₋₁₀ partly saturated and fused bicyclic hydrocarbon-C₁₋₄ alkyl group, C₉₋₁₀ partly saturated and fused bicyclic hydrocarbon-C₂₋₄ alkenyl group, C₆₋₁₀ aryl group and C₇₋₁₄ aralkyl group; and R^{21c}, R^{22c} and R^{23c} are the same or different and are an optionally substituted C₁₋₈ aliphatic hydrocarbon group, provided that the sum total of the number of carbon atoms is 7 or more),
(iii) -NR^{20c}R^{25c} (wherein R^{20c} is as defined above and R^{25c} is an optionally substituted C₆₋₁₀ aryl-C₂₋₄ alkyl, C₆₋₁₀ aryloxy-C₂₋₄ alkyl, C₆₋₁₀ arylamino-C₂₋₄ alkyl, C₇₋₁₄ aralkylamino-C₂₋₄ alkyl, 5- to 7-membered heterocyclic ring (having 1-4 hetero atoms selected from nitrogen atom, oxygen atom and sulfur atom)-C₂₋₄ alkyl or 5- to 7-membered heterocyclic group having 1-4 hetero atoms selected from nitrogen atom, oxygen atom and sulfur atom),
(iv) a substituted 5- to 7-membered cyclic amino group (e.g., piperidino, piperadino, morpholino, thiomorpholino, etc.), or
(v) -OR^{24c} (wherein R^{24c} is (a) an optionally substituted C₇₋₁₄ aralkyl group, (b) an optionally substituted C₃₋₇ alicyclic hydrocarbon group, (c) an optionally substituted C₇₋₂₄ aliphatic hydrocarbon group, or (d) an optionally substituted 5- to 7-membered aromatic or non-aromatic heterocyclic group having 1-4 hetero atoms selected from nitrogen atom, oxygen atom and sulfur atom;
wherein said substituents for R^{1a}, Z^{2c}, R^{20c}, R^{21c}, R^{22c}, R^{23c}, R^{24c} and R^{25c} are 1 to 3 substituents selected from the group consisting of
1) C₁₋₆ alkyl,
2) C₂₋₆ alkenyl,
3) C₂₋₆ alkynyl,
4) C₃₋₇ cycloalkyl,
5) C₆₋₁₀ aryl which may be substituted with 1 to 3 substituents selected from the group consisting of C₁₋₆ alkyl, amino, N-(C₁₋₆ alkyl)amino, N,N-di-(C₁₋₆ alkyl) amino, amidino, carbamoyl, N-(C₁₋₆ alkyl)carbamoyl, N,N-di-(C₁₋₆ alkyl)carbamoyl, sulfamoyl, N-(C₁₋₆ alkyl)sulfamoyl, N,N-di-(C₁₋₆ alkyl)sulfamoyl, carboxyl, C₂₋₇ alkoxycarbonyl, hydroxyl, C₁₋₆ alkoxy, mercapto, C₁₋₆ alkylthio, sulfo, cyano, azido, halogen, nitro, nitroso, phosphono, C₁₋₆ alkoxyphosphoryl, di-(C₁₋₆ alkoxy)phosphoryl and C₁₋₆ alkyl substituted with phosphono, C₁₋₆ alkoxyphosphoryl and di-(C₁₋₆ alkoxy)phosphoryl (hereinafter the group of 5) is referred to as group "C"),
6) aromatic heterocyclic group selected from (a) aromatic 5- or 6-membered heterocyclic group having 1-4 hetero atoms selected from nitrogen atom, oxygen atom and sulfur atom, (b) fused bicyclic heterocyclic group formed by condensation of an aromatic 5- or 6-membered heterocyclic group having 1 to 3 hetero atoms selected from nitrogen atom, oxygen atom and sulfur atom with benzene ring or an aromatic 5- or 6-membered heterocyclic group having 1 to 3 hetero atoms selected from nitrogen atom, oxygen atom and sulfur atom and (c) fused tricyclic heterocyclic group formed by condensation of [1] an aromatic 5- or 6-membered heterocyclic group having 1-3 hetero atoms selected from nitrogen atom, oxygen atom and sulfur atom,
   [2] benzene ring, and [3] an aromatic 5- or 6-membered heterocyclic group having 1-3 hetero atoms selected from nitrogen atom, oxygen atom and sulfur atom or benzene ring,
7) heterocyclic-oxy group formed by combining each of the above aromatic heterocyclic groups (a), (b) and (c) with oxy group,
8) non-aromatic 4- or 7-membered heterocyclic group having 1 to 3 hetero atoms selected from nitrogen atom, oxygen atom and sulfur atom,
9) C₇₋₁₄ aralkyl which may be substituted with 1 to 3 substituents selected from the group "C",
10) amino group,
11) N-mono-substituted amino selected from N-(C₁₋₆ alkyl) amino, N-(C₂₋₆ alkenyl) amino, N-(C₃₋₇ cycloalkyl) amino group and N-(C₆₋₁₀ aryl)amino which may be substituted with 1 to 3 substituents selected from the group "C",
12) amino substituted with two substituents selected from C₁₋₆ alkyl, C₂₋₆ alkenyl, C₃₋₇ cycloalkenyl and C₆₋₁₀ aryl which may be substituted with 1 to 3 substituents selected from the group "C",
13) amidino,
14) acyl selected from C₂₋₈ alkanoyl, C₃₋₈ alkenoyl, C₃₋₇ cycloalkyl-carbonyl, C₃₋₇ cycloalkenyl-carbonyl, C₆₋₁₀ aryl-carbonyl which may be substituted with 1 to 3 substituents selected from the group "C", and heterocyclic-carbonyl formed by binding of an aromatic or non-aromatic 5- or 6-membered heterocyclic group having 1-3 hetero atoms selected from nitrogen atom, oxygen atom and sulfur atom with carbonyl,
15) carbamoyl,
16) mono-substituted carbamoyl group selected from N-(C₁₋₆ alkyl) carbamoyl, N- (C₂₋₆ alkenyl) carbamoyl, N-(C₃₋₇ cycloalkyl)carbamoyl and N-(C₆₋₁₀ aryl)carbamoyl which may be substituted with 1 to 3 substituents selected from the group "CN,
17) carbamoyl substituted with two substituents selected from C₁₋₆ alkyl, C₂₋₆ alkenyl, C₃₋₇ cycloalkyl and C₆₋₁₀ aryl which may be substituted with 1 to 3 substituents selected from the group "C",
18) sulfamoyl,
19) N-mono-substituted sulfamoyl selected from N-(C₁₋₆ alkyl)sulfamoyl, N-(C₂₋₆ alkenyl) sulfamoyl, N-(C₃₋₇ cycloalkyl)sulfamoyl and N-(C₆₋₁₀ aryl) sulfamoyl which may be substituted with 1 to 3 substituents selected from the group "C",
20) sulfamoyl substituted with two substituents selected from C₁₋₆ alkyl, C₂₋₆ alkenyl, C₃₋₇ cycloalkyl and C₆₋₁₀ aryl which may be substituted with 1 to 3 substituents selected from the group "C",
21) carboxyl,
22) C₁₋₆ alkoxy-carbonyl,
23) hydroxyl,
24) C₁₋₆ alkoxy,
25) C₂₋₁₀ alkenyloxy,
26) C₃₋₇ cycloalkyloxy,
27) C₆₋₁₀ aryloxy which may be substituted with 1 to 3 substituents selected from the group "C",
28) C₇₋₁₄ aralkyloxy which may be substituted with 1 to 3 substituents selected from the group "C",
29) mercapto,
30) C₁₋₆ alkylthio,
31) C₇₋₁₄ aralkylthio which may be substituted with 1 to 3 substituents selected from the group "C",
32) C₆₋₁₀ arylthio which may be substituted with 1 to 3 substituents selected from the group "C",
33) C₁₋₆ alkylsulfinyl,
34) C₇₋₁₄ aralkylsulfinyl which may be substituted with 1 to 3 substituents selected from the group "C",
35) C₆₋₁₀ arylsulfinyl which may be substituted with 1 to 3 substituents selected from the group "C",
36) C₁₋₆ alkylsulfonyl,
38) C₇₋₁₄ aralkylsulfonyl which may be substituted with 1 to 3 substituents selected from the group "C",
39) C₆₋₁₀ arylsulfonyl which may be substituted with 1 to 3 substituents selected from the group "C",
40) sulfo,
41) cyano,
42) azido,
43) halogen,
44) nitro,
45) nitroso,
46) phosphono,
47) C₁₋₆ alkoxy-phosphoryl
48) di-C₁₋₆ alkoxy-phosphoryl,
49) C₁₋₆ alkyl substituted with phosphono, C₁₋₆ alkoxyphosphoryl or di-(C₁₋₆ alkoxy)phosphoryl
50) C₁₋₆ alkyl substituted with 1 to 4 halogen atoms
51) C₁₋₆ alkoxy substituted with 1 to 4 halogen atoms and
52) C₁₋₆ alkylenedioxy
(hereinafter the group of above 1) to 52) is referred to as group "B").

Specific examples of these substituents include the same groups as those exemplified with respect to the substituents of the 5- to 7-membered ring in ring A.

As R^{1a} of the formula (IIIa), more preferred is the group represented by the formula: -CONR^{20c}(CR^{21c}R^{22c}R^{23c}) (wherein R^{20c}, R^{21c}, R^{22c} and R^{23c} are as defined above).

Further more preferably, R^{1a} is (1) a 5- to 7-membered aromatic heterocyclic group having 1-4 hetero atoms selected from nitrogen atom, oxygen atom and sulfur atom (e.g., 1,3,4-oxadiazolyl, 1,3,4-thiadiazolyl, etc.) which is substituted with C₁₋₄ alkyl-C₇₋₁₄ aralkyl (e.g., 1-ethyl-1-(4-methylphenyl)propyl, etc.), or (2) a group represented by the formula: -CO-Z^{2c'} (wherein Z^{2c'} is
(i) -NR^{20c'}(CR^{21c'}R^{22c'}R^{23c'}) (wherein (a) R^{20c'} is a hydrogen or C₁₋₆ alkyl; R^{21c'} is a C₆₋₁₀ aryl group or a 5- to 7-membered aromatic heterocyclic group having 1-4 hetero atoms selected from nitrogen atom, oxygen atom and sulfur atom, each of which may be substituted with 1 to 3 substituents selected from the group consisting of halogen, C₁₋₆ alkyl, C₂₋₆ alkenyl, halogeno C₁₋₆ alkyl, hydroxy-C₁₋₆ alkyl, C₁₋₆ alkoxy, carboxyl, C₁₋₆ alkoxy-carbonyl, C₁₋₆ alkyl-carbonyloxy, C₁₋₆ alkyl-carbonyloxy-C₁₋₆ alkyl, carboxy-C₁₋₆ alkoxy, C₁₋₆ alkoxy-carbonyl-C₁₋₆ alkyl, C₁-₆ alkoxy-carbonyl-C₁₋₆ alkoxy, C₁₋₆ alkoxy-carbonyl-C₁₋₆ alkoxy-C₁₋₆ alkyl, carbonyl, C₁₋₆ alkyl-carbonyl, amino, mono- or di- C₁₋₆ alkylamino, phenyl (said phenyl may be substituted with 1 to 3 substituents selected from halogen, C₁₋₆ alkyl and halogeno C₁₋₆ alkyl) and a 5- to 7-membered aromatic heterocyclic group having 1-4 hetero atoms selected from nitrogen atom, oxygen atom and sulfur atom; R^{22c'} and R^{23c'} are the same or different and are C₁₋₆ alkyl group, C₅₋₇ cycloalkyl group, phenyl group (said phenyl group may be substituted with 1 to 3 substituents selected from C₁₋₆ alkyl, halogeno C₁₋₆ alkyl and C₁₋₆ alkoxy), C₁₋₆ alkoxy-carbonyl-C₁₋₆ alkyl group or C₁₋₆ alkyl-carbonyl-C₂₋₆ alkenyl group, or R^{22c'} and R^{23c'} may be combined each other to form a C₃₋₇ carbon ring; or (b) R^{20c'} and R^{21c'} are combined each other to form a 5- to 7-membered ring and said ring may be substituted with C₁₋₆ alkoxy or C₇₋₁₄ aralkyl, and R^{22c'} and R^{23c'} are C₁₋₆ alkyl group),
(ii) -NR^{20c'}R^{25c'} (wherein R^{20c'} is a hydrogen or C₁₋₆ alkyl group; R^{25c'} is C₆₋₁₀ aryl-C₂₋₄ alkyl group, C₆₋₁₀ aryloxy-C₂₋₄ alkyl group, C₆₋₁₀ arylamino-C₂₋₄ alkyl group, C₇₋₁₄ aralkylamino-C₂₋₄ alkyl group, 5- to 7-membered heterocyclic ring-C₂₋₄ alkyl group or 5- to 7-membered heterocyclic group, each of which may be substituted with 1 or 2 substituents selected from the group consisting of halogen, C₁₋₆ alkyl, C₆₋₁₀ aryl, C₁₋₆ alkoxy, amino, mono- or di- C₁₋₆ alkylamino, 5- to 7-membered cyclic amino, hydroxy, oxo, C₁₋₆ alkoxy-carbonyl and cyano), or
(iii) a 5- to 7-membered cyclic amino group which is substituted with 1 to 3 substituents selected from the group consisting of halogen, C₁₋₆ alkyl, C₂₋₆ alkenyl, C₁₋₆ alkoxy-C₁₋₆ alkyl, C₅₋₇ cycloalkyl, C₆₋₁₀ aryl (said aryl may have 1 or 2 substituents selected from halogen, C₁₋₆ alkyl, halogeno C₁₋₆ alkyl and C₁₋₆ alkoxy), C₇₋₁₄ aralkyl (said aralkyl may have 1 or 2 substituents selected from halogen, C₁₋₆ alkyl, halogeno C₁₋₆ alkyl and C₁₋₆ alkoxy), hydroxy, hydroxy-C₁₋₆ alkyl, C₆₋₁₀ aryloxy (said aryloxy may have 1 or 2 substituents selected from halogen, C₁₋₆ alkyl, halogeno C₁₋₆ alkyl and C₁₋₆ alkoxy), C₇₋₁₄ aralkyloxy, C₆₋₁₀ aryl-carbonyl, carboxyl, C₁₋₆ alkoxy-carbonyl, carbamoyl, C₆₋₁₀ aryl-carbamoyl, amino, C₆₋₁₀ aryl-carbonylamino, C₁₋₆ alkylcarbonylamino, C₁₋₆ alkoxy-carbonylamino, C₆₋₁₀ arylthio, C₆₋₁₀ arylsulfonyl, cyano, oxo and 5- to 7-membered heterocyclic group.).

R³ of the formulas (II), (III) and (IIIa) is a hydrogen, an optionally substituted hydrocarbon group, an optionally substituted hydroxyl group, an optionally substituted amino group, an optionally substituted heterocyclic group, or a group of the formula: -Z¹-Z² (wherein -Z¹- and Z² are as defined above).

Examples of the optionally substituted hydrocarbon group, the optionally substituted hydroxyl group, the optionally substituted amino group and the optionally substituted heterocyclic group of R³ include the same groups as those exemplified with respect to the substituents of the 5- to 7-membered ring in ring A.

R³ is preferably a hydrogen, a C₁₋₆ alkyl group or a C₇₋₁₄ aralkyl group, and more preferably R³ is a hydrogen.

Y in the formulas (I), (II), (III) and (IIIa) is C, CR⁴, or N.

R⁴ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above).

Examples of the optionally substituted hydrocarbon group, the optionally substituted hydroxyl group, the optionally substituted amino group, the optionally substituted heterocyclic group and halogen of R⁴ include the same groups as those exemplified with respect to the substituents of the 5- to 7-membered ring in ring A.

Y is preferably CH.

R⁸ of the formulas (III) and (IIIa) is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above).

Examples of the optionally substituted hydrocarbon group, the optionally substituted hydroxyl group, the optionally substituted amino group, the optionally substituted thiol group, halogen atom and the optionally substituted heterocyclic group of R⁸ include the same groups as those exemplified with respect to the substituents of the 5- to 7-membered ring in ring A.

R⁸ is preferably a hydrogen, a C₁₋₆ alkyl group, a C₁₋₆ alkylthio group or a C₁₋₆ alkoxy group which may be substituted with hydroxyl group, and more preferably R⁸ is a hydrogen or a C₁₋₆ alkyl group.

Ar in the formulas (I), (II), (III) and (IIIa) is an optionally substituted cyclic group.

Examples of the optionally substituted cyclic group of Ar include an optionally substituted aromatic or non-aromatic hydrocarbon ring group or an optionally substituted aromatic or non-aromatic heterocyclic group, and the like.

Examples of the aromatic hydrocarbon ring group and the heterocyclic group of Ar include the same aromatic hydrocarbon group and heterocyclic group as exemplified with respect to the substituents of the above 5- to 7-membered ring in ring A.

Examples of the non-aromatic hydrocarbon ring group include a saturated alicyclic hydrocarbon group having 3-7 carbon atoms (e.g., cycloalkyl group, etc.) such as cyclopropyl, cyclobutyl, cyclopentyl, cyclohexyl, cycloheptyl, and the like; an unsaturated alicyclic hydrocarbon group having 3-7 carbon atoms (e.g., cycloalkenyl group, cycloalkadienyl group, etc.) such as 1-cyclopentenyl, 2-cyclopentenyl, 3-cyclopentenyl, 1-cyclohexenyl, 2-cyclohexenyl, 3-cyclohexenyl, 1-cycloheptenyl, 2-cycloheptenyl, 3-cycloheptenyl, 2,4-cycloheptadienyl, etc.; a partly saturated and fused bicyclic hydrocarbon group [preferably, C₉₋₁₀ partly saturated and fused bicyclic hydrocarbon group, etc. (including those where the benzene ring is combined to 5-or 6-membered non-aromatic cyclic hydrocarbon group)] such as 1-indenyl, 2-indenyl, 1-indanyl, 2-indanyl, 1,2,3,4-tetrahydro-1-naphthyl, 1,2,3,4-tetrahydro-2-naphthyl, 1,2-dihydro-1-naphthyl, 1,2-dihydro-2-naphthyl, 1,4-dihydro-1-naphthyl, 1,4-dihydro-2-naphthyl, 3,4-dihydro-1-naphthyl, 3,4-dihydro-2-naphthyl, etc.; and the like.

Examples of the substituent of the optionally substituted aromatic ring group and the optionally substituted heterocyclic group of Ar include the same groups as those exemplified with respect to the substituents of the above 5- to 7-membered ring in ring A.

Ar is preferably (1) a C₆₋₁₀ aryl group, (2) a 5- to 7-membered aromatic or non-aromatic heterocyclic group having 1-4 hetero atoms selected from nitrogen atom, oxygen atom and sulfur atom, or (3) a C₃₋₇ saturated or unsaturated alicyclic hydrocarbon group, each of which may be substituted with 1 to 3 substituents selected from the group "B".

More preferably, Ar is a C₆₋₁₀ aryl group which may be substituted with 1 to 3 substituents selected from the group "B", a 5- to 7-membered aromatic heterocyclic group having 1-4 hetero atoms selected from nitrogen atom, oxygen atom and sulfur atom which may be substituted with 1 to 3 substituents selected from the group "B", or a C₃₋₇ saturated or unsaturated alicyclic hydrocarbon group.

Further more preferably, Ar is (1) a C₆₋₁₀ aryl group (e.g., phenyl, naphthyl, etc.) which may be substituted with 1 or 2 substituents selected from the group consisting of halogen atom, C₁₋₆ alkyl, C₁₋₆ alkoxy, hydroxy, C₇₋₁₄ aralkyloxy and mono- or di-C₁₋₄ alkylamino, (2) a 5- to 7-membered aromatic heterocyclic group having 1-4 hetero atoms selected from nitrogen atom, oxygen atom and sulfur atom (e.g., pyridyl, furyl, thiazolyl, thienyl, etc.) which may be substituted with C₁₋₄ alkyl or (3) a C₅₋₇ cycloalkyl group (e.g., cyclohexyl etc.), and most preferably, Ar is an optionally halogenated phenyl group.

R⁹ and R¹⁰ of the formulas (II), (III) and (IIIa) are the same or different and are a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above), or R⁹ and R¹⁰ may be combined to form an oxo group, methylene group or a ring.

Examples of the optionally substituted hydrocarbon group, the optionally substituted hydroxyl group, the optionally substituted amino group, the optionally substituted thiol group, a halogen atom and the optionally substituted heterocyclic group of R⁹ and R¹⁰ include the same groups as those exemplified with respect to the substituents of the above 5- to 7-membered ring in ring A.

One of R⁹ and R¹⁰ is preferably a hydrogen atom or C₁₋₆ alkyl group which may be substituted with 1 to 3 substituents selected from the group "B" and the other is (1) a hydrocarbon group selected from C₁₋₈ saturated aliphatic hydrocarbon group, C₂₋₈ unsaturated aliphatic hydrocarbon group, C₃₋₇ saturated alicyclic hydrocarbon group, C₃₋₇ unsaturated alicyclic hydrocarbon group, C₉₋₁₀ partly saturated and fused bicyclic hydrocarbon group, C₃₋₇ saturated or unsaturated alicyclic-C₁₋₈ saturated or unsaturated aliphatic hydrocarbon group, C₉₋₁₀ partly saturated and fused bicyclic hydrocarbon-C₁₋₄ alkyl group, C₉₋₁₀ partly saturated and fused bicyclic hydrocarbon-C₂₋₄ alkenyl group, C₆₋₁₀ aryl group and C₇₋₁₄ aralkyl group, each of which may be substituted with 1 to 3 substituents selected from the group "B" or (2) a 5- to 7-membered aromatic or non-aromatic heterocyclic group having 1-4 hetero atoms selected from nitrogen atom, oxygen atom and sulfur atom, which may be substituted with 1 to 3 substituents selected from the group "B", or R⁹ and R¹⁰ may be combined to form a C₅₋₇ carbon ring.

More preferably, one of R⁹ and R¹⁰ is preferably a hydrogen atom or C₁₋₆ alkyl group and the other is an optionally halogenated C₁₋₆ alkyl group, C₆₋₁₀ aryl group, C₇₋₁₀ aralkyl group or a 5- to 7-membered aromatic heterocyclic group, or R⁹ and R¹⁰ are a C₅₋₇ carbon ring formed by combining together.

R¹¹ and R¹² of the formula (II) are the same or different and are a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z^{1'}-Z² (wherein -Z^{1'}- is -CS-, -SO- or -SO₂-, and Z² is as defined above).

Examples of the optionally substituted hydrocarbon group, the optionally substituted hydroxyl group, the optionally substituted amino group, the optionally substituted thiol group, a halogen atom and the optionally substituted heterocyclic group of R¹¹ and R¹² include the same groups as those exemplified with respect to the substituents of the above 5- to 7-membered ring in ring A.

R⁹ and R¹⁰, or R¹¹ and R¹² may be combined to form an oxo group, methylene group or a ring such as a C₃₋₆ saturated or unsaturated carbon ring (e.g., cyclopropyl, cyclobutyl, cyclopentyl, cyclohexyl, 1-cyclopentenyl, 2-cyclopentenyl, 3-cyclopentenyl, 1-cyclohexenyl, 2-cyclohexenyl, 3-cyclohexenyl, etc.); or R¹⁰ and R¹¹ may be combined to form a ring such as a C₃₋₆ saturated or unsaturated carbon ring (e.g., cyclopropyl, cyclobutyl, cyclopentyl, cyclohexyl, 1-cyclopentenyl, 2-cyclapentenyl, 3-cyclopentenyl, 1-cyclohexenyl, 2-cyclohexenyl, 3-cyclohexenyl, etc.).

----- is a single bond or a double bond.

Z in the formula (I) is CR⁵, CR⁵R⁶, N or NR⁷, and CR⁵ is as defined above.

R⁶ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above).

Examples of the optionally substituted hydrocarbon group, the optionally substituted hydroxyl group, the optionally substituted amino group, the optionally substituted thiol group, and the optionally substituted heterocyclic group of R⁶ include the same groups as those exemplified with respect to the substituents of the above 5- to 7-membered ring in ring A.

R⁷ is a hydrogen, an optionally substituted hydrocarbon group, an optionally substituted hydroxyl group, an optionally substituted amino group, cyano group, a halogen atom, an optionally substituted heterocyclic group, or a group of the formula: -Z¹-Z² (wherein -Z¹- and Z² are as defined above)).

Examples of the optionally substituted hydrocarbon group, the optionally substituted hydroxyl group, the optionally substituted amino group, a halogen atom and the optionally substituted heterocyclic group of R⁷ include the same groups as those exemplified with respect to the substituents of the above 5- to 7-membered ring in ring A.

R⁵, R⁶ and R⁷ may be the same or different.

R⁵ is a hydrogen, an optionally substituted hydrocarbon group, an optionally substituted hydroxyl group, an optionally substituted amino group, an optionally substituted thiol group, an optionally substituted sulfonyl group or an optionally substituted sulfinyl group.

Examples of the optionally substituted hydrocarbon group, the optionally substituted hydroxyl group, the optionally substituted amino group, the optionally substituted thiol group, the optionally substituted sulfonyl group and the optionally substituted sulfinyl group of R include the same groups as those exemplified with respect to the substituents of the above 5- to 7-membered ring in ring A.

R and Z may be combined to form a ring B

Ring B in the formula (I) is an optionally substituted 5- to 7-membered heterocyclic ring and examples thereof include the same group as that exemplified with respect to the 5- to 7-membered ring of ring A.

X² of the formula (I) is N or NR³, and R³ is as defined above.

X³ of the formulas (III) and (IIIa) is a bond, oxygen atom, an optionally oxidized sulfur atom, N, NR^{7'}, or an optionally substituted bivalent C₁₋₂ hydrocarbon group.

R^{7'} is a hydrogen, an optionally substituted hydrocarbon group, an optionally substituted hydroxyl group, an optionally substituted amino group, an optionally substituted heterocyclic group, or a group of the formula - Z^{1'}-Z² (wherein -Z^{1'}- is -CS-, -SO- or -SO₂-, and Z² is as defined above).

Examples of the optionally substituted hydrocarbon group, the optionally substituted hydroxyl group, the optionally substituted amino group, and the optionally substituted heterocyclic group of R^{7'} include the same groups as those exemplified with respect to the substituents of the above 5- to 7-membered ring in ring A.

Examples of the optionally substituted bivalent C₁₋₂ hydrocarbon group include -CH₂-, -(CH₂)₂-, -CH=CH- and the like which may be substituted with one or two substituents selected from those exemplified with respect to the substituents of the above 5- to 7-membered ring in ring A.

In the formula (IIIa), X³ is preferably CH₂.

Prefered compounds of the formula (I) include not only the compounds of the formula (IIIa) but also the other compounds wherein -Z¹- is -CO- and Z² is an optionally substituted hydroxyl group (e.g., hydroxy, C₁₋₆ alkoxy, etc.) or amino group which is substituted with an optionally substituted phenyl group or an optionally substituted condensed phenyl group (e.g., phenylamino, 3,5-dimethoxyphenylamino, 3-biphenylylamino, 2,3-dihydro-1H-inden-5-yl-amino, quinolin-6-yl-amino, etc.).

In the formula (II),
(1) when ring A is a 6-membered ring and Q is C or CR⁵, X¹ is C-Z¹-Z², C(-Z¹-Z²)R² or N-Z¹-Z², and neither R⁹ nor R¹⁰ is a hydrogen, or R⁹ and R¹⁰ are not combined to form an oxo group, or R¹⁰ and R¹¹ are not combined to form a 5-membered ring;
(2) when ring A is a 6-membered ring and Q is N, X¹ is C-Z¹-Z², C(-Z¹-Z²)R² or N-Z¹-Z², and R⁹ and R¹⁰ are not combined to form an oxo group;
(3) when ring A is a 5-membered ring and Q is C or CR⁵, X¹ is C-Z¹-Z², C(-Z¹-Z²)R² or N-Z¹-Z², and Z² is an optionally substituted amino group; and
(4) when ring A is a 5-membered ring and Q is N, at least one of R⁹ and R¹⁰ is CHR¹⁵R¹⁶ (wherein R¹⁵ and R¹⁶ are the same or different and are a hydrogen, an optionally substituted hydrocarbon group, an optionally substituted hydroxyl group, an optionally substituted amino group, an optionally substituted thiol group, a halogen atom, an optionally substituted heterocyclic group, or a group of the formula:-Z¹-Z² (wherein -Z¹- and Z² are as defined above).

Examples of the optionally substituted hydrocarbon group, the optionally substituted hydroxyl group, the optionally substituted amino group, the optionally substituted thiol group, halogen atom and the optionally substituted heterocyclic group include the same groups as those exemplified with respect to the substituents of the above 5- to 7-membered ring in ring A.

In the formulas (II) and (III), preferably, R¹ is a group of the formula: -Z¹-Z²; Z¹ is -CO- and Z² is an optionally substituted hydroxyl group or an optionally substituted amino group; Ar is an optionally substituted aromatic ring group; and both R⁹ and R¹⁰ are the same or different and are C₁₋₆ alkyl groups or R⁹ and R¹⁰ are combined to form a ring such as a saturated or unsaturated C₃₋₆ ring as described above.

In the formula (III), preferably, R³ is a hydrogen. More preferably, in the formula (III), R¹ is a group of the formula: -Z¹-Z² (wherein -Z¹- is -CO-, -CS-, -SO- or -SO₂-, and Z² is an optionally substituted hydrocarbon group, an optionally substituted heterocyclic group, an optionally substituted hydroxyl group, or an optionally substituted amino group); R³ is a hydrogen; Ar is an optionally substituted aromatic ring group; X³ is CR¹¹R¹² (wherein R¹¹ and R¹² are the same or different and are a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above), or R¹¹ and R¹² may be combined to form an oxo group, methylene group or a ring such as a saturated or unsaturated C₃₋₆ ring as described above); and R⁹ and R¹⁰ are the same or different and are a C₁₋₆ alkyl group, or R⁹ and R¹⁰ may be combined to form a ring such as a saturated or unsaturated C₃₋₆ ring as described above.

As preferable compounds of formulas (I), (II), (III) or (IIIa), exemplified are:
N-(1-ethyl-1-(4-methylphenyl)propyl)-7,7-dimethyl-5-phenyl-4,5,6,7-tetrahydropyrazolo[1,5-a]pyrimidine-3-carboxamide or a salt thereof,
N-(1-ethyl-1-(4-methylphenyl)propyl)-5-(2-fluorophenyl)-7,7-dimethyl-4,5,6,7-tetrahydropyrazolo[1,5-a]pyrimidine-3-carboxamide or a salt thereof,
N-(1-ethyl-1-(4-methylphenyl)propyl)-2,7,7-trimethyl-5-phenyl-4,5,6,7-tetrahydropyrazolo[1,5-a]pyrimidine-3-carboxamide or a salt thereof,
N-(1-ethyl-1-(4-ethylphenyl)propyl)-2,7,7-trimethyl-5-phenyl-4,5,6,7-tetrahydropyrazolo[1,5-a]pyrimidine-3-carboxamide or a salt thereof,
N-(1-ethyl-1-(4-methylphenyl)propyl)-5-(2-fluorophenyl)-2,7,7-trimethyl-4,5,6,7-tetrahydropyrazolo[1,5-a]pyrimidine-3-carboxamide or a salt thereof,
N-(1-ethyl-1-(4-ethylphenyl)propyl)-5-(2-fluorophenyl)-2,7,7-trimethyl-4,5,6,7-tetrahydropyrazolo[1,5-a]pyrimidine-3-carboxamide or a salt thereof,
5-(2-chlorophenyl)-N-(1-ethyl-1-(4-methylphenyl)propyl)-2,7,7-trimethyl-4,5,6,7-tetrahydropyrazolo[1,5-a]pyrimidine-3-carboxamide or a salt thereof,
N-(1-(4-(dimethylamino)phenyl)-1-ethylpropyl)-5-(2-fluorophenyl)-2,7,7-trimethyl-4,5,6,7-tetrahydropyrazolo[1,5-a]pyrimidine-3-carboxamide or a salt thereof,
N-(1,1-diethylbutyl)-5-(2-fluorophenyl)-2,7,7-trimethyl-4,5,6,7-tetrahydropyrazolo[1,5-a]pyrimidine-3-carboxamide or a salt thereof,
N-(1-ethyl-1-phenylpropyl)-5-(2-fluoropheny)-2,7,7-trimethyl-4,5,6,7-tetrahydropyrazolo[1,5-a]pyrimidine-3-carboxamide or a salt thereof,
3-(5-(1-ethyl-1-(4-methylphenyl)propyl)-1,3,4-oxadiazol-2-yl)-2,7,7-trimethyl-5-phenyl-4,5,6,7-tetrahydropyrazolo[1,5-a]pyrimidine or a salt thereof,
3-(5-(1-ethyl-1-(4-methylphenyl)propyl)-1,3,4-thiadiazol-2-yl)-2,7,7-trimethyl-5-phenyl-4,5,6,7-tetrahydropyrazolo[1,5-a]pyrimidine or a salt thereof,
3-((4-(benzyloxy)-2,2-diethyl-1-pyrrolidinyl)carbonyl)-7,7-dimethyl-5-phenyl-4,5,6,7-tetrahydropyrazolo[1,5-a]pyrimidine or a salt thereof,
3-((2,2-diethyl-4-methoxy-1-pyrrolidinyl)carbonyl)-7,7-dimethyl-5-phenyl-4,5,6,7-tetrahydropyrazolo[1,5-a]pyrimidine or a salt thereof, or
3-((2,2-diethyl-4-fluoro-1-pyrrolidinyl)carbonyl)-7,7-dimethyl-5-phenyl-4,5,6,7-tetrahydropyrazolo[1,5-a]pyrimidine or a salt thereof; and the like.

As a salt of the compound of formula (I), (II), (III) or (IIIa) (hereinafter sometimes referred to as Compound (I), (II), (III) or (IIIa)), a pharmaceutically acceptable salt is preferred. Examples thereof include a salt with an inorganic base, a salt with an organic base, a salt with an inorganic acid, a salt with an organic acid, a salt with a basic or acidic amino acid, or the like. Preferred examples of the salt with an inorganic base include an alkali metal salt such as sodium salt, potassium salt, or the like; an alkaline earth metal salt such as calcium salt, magnesium salt, or the like; and aluminum salt; ammonium salt; or the like. Preferred examples of the salt with an organic base include a salt with trimethylamine, triethylamine, pyridine, picoline, ethanolamine, diethanolamine, triethanolamine, dicyclohexylamine, N,N'-dibenzylethylenediamine, or the like. Preferred examples of the salt with an inorganic acid include a salt with hydrochloric acid, hydrobromic acid, nitric acid, sulfuric acid, phosphoric acid, or the like. Preferred examples of the salt with an organic acid include a salt with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, or the like. Preferred examples of the salt with a basic amino acid include a salt with arginine, lysine, ornithine or the like. Preferred examples of the salt with an acidic amino acid include a salt with aspartic acid, glutamic acid, or the like.

Compound (I), (II), (III) or (IIIa) may be in the form of a prodrug thereof. The prodrug of Compound (I), (II), (III) or (IIIa) refers to a compound that is converted into Compound (I), (II), (III) or (IIIa) by a reaction with an enzyme, gastric acid, or the like under a physiological condition in the living body, namely, (i) a compound that is converted into Compound (I), (II), (III) or (IIIa) by an enzymatic oxidation, reduction, hydrolysis, or the like, and (ii) a compound that is converted into Compound (I), (II), (III) or (IIIa) by hydrolysis with gastric acid or the like. Examples of the prodrug of Compound (I), (II), (III) or (IIIa) to be used include a compound or its salt wherein hydroxyl group in Compound (I), (II), (III) or (IIIa) is acylated, alkylated, phosphorylated, or converted into borate (e.g., a compound or its salt wherein hydroxyl group in Compound (I), (II), (III) or (IIIa) is converted into acetyloxy, palmitoyloxy, propanoyloxy, pivaloyloxy, succinyloxy, fumaryloxy, alanyloxy, dimethylaminomethylcarbonyloxy, etc.), a compound or its salt wherein carboxyl group in Compound (I), (II), (III) or (IIIa) is esterified or amidated (e.g., a compound or its salt wherein carboxyl group in Compound (I), (II), (III) or (IIIa) is subjected to ethyl esterification, phenyl esterification, carboxyoxymethyl esterification, dimethylaminomethyl esterification, pivaloyloxymethyl esterification, ethoxycarbonyloxyethyl esterification, phthalidyl esterification, (5-methyl-2-oxo-1,3-dioxolan-4-yl)methyl esterification, cyclohexyloxycarbonyl esterification, or conversion into the methyl amide, etc.), or the like. These prodrugs can be produced according to a per se known method or its modified method.

Further, the prodrug of Compound (I), (II), (III) or (IIIa) may be a compound or its salt that is converted into Compound (I), (II), (III) or (IIIa) under physiological conditions as described in "Development of Drugs", Volume 7, Molecular Design, Hirokawa Shoten, 1990; pages 163-198.

Compound (I), (II), (III) or (IIIa) may be labeled with an isotope (for example, ²H, ³H, ¹⁴C, ³⁵S, ¹²⁵I, or the like) or the like.

When the compound obtained by the present invention or a salt thereof has a double bond in its molecule and a steric configuration of Z or E exsits, each of the stereoisomers and a mixture thereof are included in the present invention.

When a steric configuration exsits due to an asymmetric carbon in the molecule of the compound obtained by the present invention or a salt thereof, each of them and a mixture thereof are included in the present invention.

The Compound (I), (II), (III) or (IIIa) can be produced according to the method described in WO 2005/00502.

Compound (I), (II), (III) or (IIIa) has an excellent Ca receptor modulating activity and enhances the secretion of PTH, and therefore useful as drugs for treating bone diseases, kidney-acting drugs, central nervous system and endocrine-acting drugs, digestive system-acting drugs, and the like. Further, the toxicity is low. Therefore, Compound (I), (II), (III) or (IIIa) may be safely administered to mammalian animals (for example, human, rat, mouse, dog, rabbit, cat, cow, horse, pig, and the like).

Thus, a pharmaceutical composition containing Compound (I), (II), (III) or (IIIa) is expected to be useful in the treatment and prevention of diseases, in which Ca receptor modulating activity is required, such as
Ca receptor modulating drugs: primary or secondary hyper parathyroidism; hypoparathyroidism; hyperthyroidism; hypothyroidism; Graves' disease; Hashimoto's toxicosis; Paget's disease; hypercalcemia associated with malignant tumor; hypercalcemia; hypocalcemia; postmenopausal osteoporosis; senile osteoporosis; secondary osteoporosis; osteomalacia; renal osteodystrophy; fracture; osteoarthritis; rheumatoid arthritis; osteosarcoma; myeloma; hypertension; diabetes; myocardial infarction; Hachington's diseases; Parkinson's diseases; Alzheimer's disease; dementia; cerebral apoplexy; brain tumor; spinal injury; diabetic renal disease; renal insufficiency; gastric ulcer; duodenal ulcer; Basedow's disease; parathyroid gland tumor; thyride gland tumor; arteriosclerosis; and the like;
Ca receptor antagonistic drugs: hyperthyroidism; hypocalcemia; postmenopausal osteoporosis; senile osteoporosis; secondary osteoporosis; osteomalacia; renal osteodystrophy; fracture; osteoarthritis; rheumatoid arthritis; osteosarcoma; myeloma; central nervous system diseases; and the like, in particular osteoporosis.

The dosage of Compound (I), (II), (III) or (IIIa) can be selected in various ways depending on the administration route and the symptom of a patient to be treated. The dosage of Compound (I), (II), (III) or (IIIa) per an adult (a body weight of 50 kg) can be usually selected in a range of about 0.1 mg to about 2,000 mg, preferably about 0.1 mg to about 500 mg, further preferably about 1 mg to about 300 mg in the case of oral administration and in a range of about 0.01 mg to about 100 mg, further preferably about 0.1 mg to about 10 mg in the case of parenteral administration. The dosage can be administered with being divided in 1 to 3 times daily.

In addition, Compound (I), (II), (III) or (IIIa) can be used in Active stage of ADFR (Active-Depress-Free-Repeat) therapy. (ADFR therapy: a therapy expecting increase in new bone by, in a short period of resting stage in bone remodeling: resting stage active stage → resorption stage → reversal stage → formation stage, 1) stimulating bone resorption (Active), 2) suppressing bone resorption by emerged osteoclasts (Depress), 3) promoting bone formation by releasing bone formation action by osteoblasts from inhibition (Free), and repeating this process)

Therefore, the drug comprising a combination of Compound (I), (II), (III) or (IIIa) and a concomitant drug described below has an excellent Ca receptor modulating activity, Ca receptor antagonistic action and the like, and is less toxic, and has few side effect, thus it is useful as a safe medicine, Ca receptor modulator, Ca receptor antagonist and the like.

The drug comprising a combination of Compound (I), (II), (III) or (IIIa) and a concomitant drug described below exhibits an excellent Ca receptor modulating activity, Ca receptor antagonistic action and the like to a mammal (for example, mouse, rat, hamster, rabbit, cat, dog, cow, sheep, monkey, human, and the like), and excels in (oral) absorbability, (metabolic) stability and the like, thus it can be used as a prophylactic and/or therapeutic agent for primary or secondary hyper parathyroidism; hypoparathyroidism; hyperthyroidism; hypothyroidism; Graves' disease; Hashimoto's toxicosis; Paget's disease; hypercalcemia associated with malignant tumor; hypercalcemia; hypocalcemia; postmenopausal osteoporosis; senile osteoporosis; secondary osteoporosis; osteomalacia; renal osteodystrophy; fracture; osteoarthritis; rheumatoid arthritis; osteosarcoma; myeloma; hypertension; diabetes; myocardial infarction; Hachington's diseases; Parkinson's diseases; Alzheimer's disease; dementia; cerebral apoplexy; brain tumor; spinal injury; diabetic renal disease; renal insufficiency; gastric ulcer; duodenal ulcer; Basedow's disease; parathyroid gland tumor; thyride gland tumor; arteriosclerosis; hyperthyroidism; hypocalcemia; postmenopausal osteoporosis; senile osteoporosis; secondary osteoporosis; osteomalacia; renal osteodystrophy; fracture; osteoarthritis; rheumatoid arthritis; osteosarcoma; myeloma; central nervous system diseases; and the like, in particular osteoporosis.

As the drug that can be used together with Compound (I), (II), (III) or (IIIa) (hireinafter, sometimes abbreviated as concomitant drug), following drugs are exemplified.

### (1) Therapeutic agent for diabetes

Insulin preparations [e.g., animal Insulin preparations extracted from cattle, pig pancreas; human Insulin preparations synthesized by genetic engineering using Escherichia coli or yeast; Insulin zinc; Protamineinsulin zinc; fragment or derivative of Insulin (e.g., INS-1 etc.) etc.], Insulin sensitive potentiators (e.g., pioglitazone hydrochloride, troglitazone, rosiglitazone or maleate thereof, JTT-501, MCC-555, YM-440, GI-262570, KRP-297, FK-614, CS-011 etc.), α-Glucosidase inhibitors (e.g., voglibose, acarbose, miglitole, emiglitate etc.), biguanide agents (e.g., phenformin, metformin, buformin etc.), sulfonylurea preparations (e.g., tolbutamide, glibenclamide, gliclazid, chloropropamide, tolazamide, acetohexamide, glyclopyramide, glimepiride etc.) and other Insulin secretion accelerating drugs (e.g., repaglinide, senaglinide, mitiglinide or its calcium salt hydrate, GLP-1, nateglinide etc.), dipeptidyl-peptidase IV inhibitors (e.g., NVP-DPP-278, PT-100, P32/98 etc.), β3 agonists (e.g., CL-316243, SR-58611-A, UL-TG-307, AJ-9677, AZ40140 etc.), amylin agonists (e.g., pramlintide etc.), phosphotyrosine phosphatase inhibitors (e.g., vanadic acid etc.), gluconeogenic inhibitors (e.g., glycogen phosphorylase inhibitor, glucose-6-phosphatase inhibitor, glucagon antagonist etc.), SGLT (sodium-glucose cotransporter) inhibitors (e.g., T-1095 etc.), and the like.

### (2) Therapeutic agent for diabetic complication

aldose reductase inhibitors (e.g., tolrestat, epalrestat, zenarestat, zopolrestat, fidarestat (SNK-860), minalrestat (ARI-509), CT-112 etc.), neurotrophic factor (e.g., NGF, NT-3 etc.), AGE inhibitors (e.g., ALT-945, pimagedine, pyratoxanthine, N-phenacylthiazolium bromide (ALT-766), EXO-226 etc.), active oxygen removers (e.g., thioctic acid etc.), cerebrovascular dilators (e.g., tiapride, etc.), and the like.

### (3) Antilipemic drug

statin compounds that are cholesterol biosynthesis inhibitor (e.g., pravastatin, simvastatin, lovastatin, atorvastatin, fluvastatin, cerivastatin, or a salt thereof (e.g., sodium salt etc.) etc.), squalene synthase inhibitors or fibrate compounds having triglyceride lowering activity (e.g., bezafibrate, clofibrate, simfibrate, clinofibrate, etc.), and the like.

### (4) Antihypertensive

angiotensin-converting enzyme inhibitors (e.g., captopril, enalapril, delapril, etc.), angiotensin II antagonists (e.g., losartan, candesartan cilexetil, etc.), calcium antagonists (e.g., manidipine, nifedipine, amlodipine, efonidipine, nicardipine, etc.), clonidine and the like.

### (5) Anti-obesity agent

central anti-obesity drug (e.g., dexfenfluamine, fenfluramine, phentermine, sibutramine, amfepramone, dexamfetamine, mazindol, phenylpropanolamine, clobenzorex etc.), pancreatic lipase inhibitors (e.g., orlistat etc.), β3 agonists (e.g., CL-316243, SR-58611-A, UL-TG-307, AJ-9677, AZ40140 etc.), peptidic appetite suppressants (e.g., leptin, CNTF (ciliary neurotrophic factor) etc.), cholecystokinin antagonists (e.g., lintitript, FPL-15849 etc.), and the like.

### (6) Diuretic drug

xanthine derivatives (e.g., sodiumu salicylate theobromine, calcium salicylate theobromine, etc.), thiazide preparations (e.g., ethiazide, cyclopenthiazide, trichlormethiazide, hydrochlorothiazide, hydroflumethiazide, benzylhydrochlorothiazide, penflutiazide, polythiazide, methyclothiazide, etc.), aldosterone antagonists (e.g., spironolactone, triamterene etc.), carbonic anhydrase inhibitors (e.g., acetazolamide, etc.), chlorobenzenesulfonamide preparations (e.g., chlortalidone, mefruside, indapamide, etc.), azosemide, isosorbide, ethacrynic acid, piretanide, bumetanide, furosemide, and the like.

### (7) Chemotherapeutic agent

alkylating agents (e.g., cyclophosphamide, ifosfamide, etc.), antimetabolites (e.g., methotrexate, 5-fluorouracil, etc.), anticancer antibiotics (e.g., mitomycin, adriamycin, etc.), anticancer drugs derived from plant (e.g., vincristine, vindesine, Taxol, etc.), cisplatin, carboplatin, etopoxide, etc., in particular, Furtulon (derivative of 5-fluorouracil) or neofurtulon and the like.

### (8) Immunotherapeutic agent

ingredients of microorganism or bacteria (e.g., muramyldipeptide derivative, Picibanil, etc.), polysaccharides having immunopotentiation activity (e.g., lentinan, sizofiran, Krestin, etc.), cytokines obtained by genetic engineering method (e.g., interferon, interleukin (IL), etc.), colony-stimulating factors (e.g., granulocyte colony-stimulating factor, erythropoietin, etc.), in particular, IL-1, IL-2, IL-12 and the like.

### (9) Drugs wherein cachectic improvement action is

acknowledged in animal model or clinical test progesteron derivatives (e.g., megesterol acetate)[ Journal of Clinical Oncology, Vol. 12, pages 213-225, 1994], metoclopramide agent, tetrahydrocannabinol agent (reference is the same as the above-described, respectively), lipid metabolism improving agent (e.g., eicosapentaenoic acid etc.) [British Journal of Cancer, Vol. 68, pages 314-318, 1993], growth hormone, IGF-1, or antibodies against TNF-α, LIF, IL-6, or oncostatin M which are factors inducing cachexia, and the like.

### (10) Antiphlogistics

steroidal drugs (e.g., dexamethasone, etc.), sodium hyaluronate, cyclooxygenase inhibitors (e.g., indomethacin, ketoprofen, loxoprofen, meloxicam, ampiroxicam, celecoxib, rofecoxib etc.), and the like.

### (11) Prophylactic or therapeutic agent for climacteric disorder

estrogen, selective estrogen receptor modulators (SERM) (e.g., raloxifene etc.)

### (12) Bone resorption inhibitor

estrogen, selective estrogen receptor modulators (SERM) (e.g., raloxifene etc.), RANKL inhibitors (e.g., AMG-162 etc.), calcitonin, active vitamin D3, vitamin K2, isoflavone preparations (e.g., ipriflavone), vitronectin receptor antagonists, V-H+-ATPase inhibitors, Src kinase inhibitors, cathepsin K inhibitors (e.g., AAE581, 462795 etc.), bisphosphonates, and the like.

### (13) Bone formation accelerator

PTH preparations, strontium and the like

### (14) Calcium preparation

### (15) LH-RH analogue

Leuplin and the like

### (16) Others

glycation inhibitors (e.g., ALT-711 etc.), nerve regeneration accelerators (e.g., Y-128, VX853, prosaptide etc.), central nervous system agents (e.g., antidepressants such as desipramine, amitriptyline, imipramine, fluoxetine, paroxetine, doxepin, duloxetine, venlafaxine), antiepileptic drugs (e.g., lamotrigine, carbamazepine, gavapentin), antiarrhythmic drugs (e.g., mexiletine), acetylcholine receptor ligands (e.g., ABT-594), endothelin receptor antagonists (e.g., ABT-627), monoamine uptake inhibitors (e.g., tramadol), indoleamine uptake inhibitors (e.g., fluoxetine, paroxetine), narcotic analgesics (e.g., morphine), non-narcotic analgesics (e.g., buprenorphine, axomadol), GABA receptor agonists, GABA uptake inhibitors (e.g., tiagabine), α₂ receptor agonists (e.g., clonidine), local analgesics (e.g., capsaicin), protein kinase C inhibitors (e.g., LY-333531), antianxietys (e.g., benzodiazepines), phosphodiesterase inhibitors (e.g., sildenafil), dopamine receptor agonists (e.g., apomorphine), dopamine receptor antagonists (e.g., haloperidol), serotonin receptor agonists (e.g., tandospirone citrate, sumatriptan, tegaserod), serotonin receptor antagonists (e.g., cyproheptadine hydrochloride, ondansetron), serotonin uptake inhibitors (e.g., fluvoxamine maleate, fluoxetine, paroxetine), sleep-inducind drugs (e.g., triazolam, zolpidem), anticholinergics (e.g., atropine, scopolamine etc.), α₂ receptor blockers (e.g., tamsulosin, urapidil, naftopidil), muscle relaxants (e.g., baclofen etc.), potassium channnel openers (e.g., nicorandil), calcium channnel blockers (e.g., nifedipine) chloride channnel openers (e.g., lubiprostone), prophylactic or therapeutic drugs for Alzheimer disease (e.g., donepezil, rivastigmine, galantamine), therapeutic drugs for Parkinson disease (e.g., L-dopa), prophylactic or therapeutic drugs for multiple sclerosis (e.g., interferon β-1a), histamine H₁ receptor inhibitors (e.g., promethazine hydrochloride), proton pump inhibitors (e.g., lansoprazole, omeprazole), antithrombotic drugs (e.g., aspirin, cilostazol), NK-2 receptor antagonists (e.g., GR159897, GR149861, SR48968 (saredutant), etc.), NK-3 receptor antagonists (e.g., talnetant), therapeutic drugs for HIV infection (e.g., saquinavir, zidovudine, lamivudine, nevirapine), therapeutic drugs for chronic obstructive pulmonary disease (salmeterol, tiotropium bromide, cilomilast), diuretics (e.g., furosemide), antidiuretic agents (e.g., vasopressin V2 receptor agonists, etc.), aromatase inhibitors (e.g., fadrozole hydrochloride, anastrozole, letrozole, exemestane, vorozole, formestane, etc,) and the like.

As the concomitant drugs in the present invention, preferred are prophylactic or therapeutic agent for climacteric disorder, bone resorption inhibitor, bone resorption accelerator, calcium preparation, LH-RH analogue and the like, and among them, bone resorption inhibitor (estrogen, selective estrogen receptor modulators (SERM) (e.g., raloxifene etc.), RANKL inhibitors (e.g., AMG-162 etc.), calcitonin, active vitamin D3, vitamin K2, isoflavone preparations (e.g., ipriflavone), vitronectin receptor antagonists, V-H+-ATPase inhibitors, Src kinase inhibitors, cathepsin K inhibitors (e.g., AAE581, 462795 etc.)) is preferred.

By combining Compound (I), (II), (III) or (IIIa) and the concomitant drug, excellent effects such as
(1) the dose can be reduced compared to the case when Compound (I), (II), (III) or (IIIa), or the concomitant drug is administered alone, respectively,
(2) the drug used in combination with Compound (I), (II), (III) or (IIIa) can be selected depending on the symptom of the patient (mild symptom, severe symptom, etc.),
(3) the treatment can be set over a long period of time by selecting concomitant drug having an action mechanism different from that of Compound (I), (II), (III) or (IIIa),
(4) treatment effects can be sustained by selecting concomitant drug having an action mechanism different from that of Compound (I), (II), (III) or (IIIa),
(5) synergistic effects can be obtained by using Compound (I), (II), (III) or (IIIa) in combination with the concomitant drug, and the like can be obtained.

Herein, the drug comprising a combination of Compound (I), (II), (III) or (IIIa) and the concomitant drug is sometimes referred to as "combined drug of the present invention".

When the combined drug of the present invention is used, the timing of administration of Compound (I), (II), (III) or (IIIa) and the concomitant drug is not limited particularly, and Compound (I), (II), (III) or (IIIa) or a pharmaceutical composition thereof and the concomitant drug or a pharmaceutical composition thereof may be administered to the administration subject simultaneously or at a time interval. In addition, the combined drug of the present invention can be used after synovectomy, after treatment using Prosorba column, after using mononuclear cell therapy and the like. The dosage of the concomitant drug can be determined according to the dose clinically used, and selected appropriately by taking into consideration of the subject to be administered, administration route, disease to be treated, the combination thereof and the like.

The administration mode of the combined drug of the present invention is not limited particularly as long as Compound (I), (II), (III) or (IIIa) and the concomitant drug are combined upon administration. For example, such an administration mode includes (1) administration of a single preparation obtained by formulating Compound (I), (II), (III) or (IIIa) and the concomitant drug simultaneously, (2) simultaneous administration of two kinds of preparations obtained by formulating Compound (I), (II), (III) or (IIIa) and the concomitant drug separately, via the same administration route, (3) separate administration at a time interval of two kinds of preparations obtained by formulating Compound (I), (II), (III) or (IIIa) and the concomitant drug separately, via the same administration route, (4) simultaneous administration of two kinds of preparations obtained by formulating Compound (I), (II), (III) or (IIIa) and the concomitant drug separately, via different administration routes, and (5) separate administration at a time interval of two kinds of preparations obtained by formulating Compound (I), (II), (III) or (IIIa) and the concomitant drug separately, via different administration routes (e.g. administration of Compound (I), (II), (III) or (IIIa), followed by the concomitant drug; or administration in the reverse order).

The combined drug of the present invention is low toxic, and can safely be administered orally or parenterally as a pharmaceutical composition such as tablets (including a sugar-coated tablet, a film coating tablet), powder, granules, capsules (including a soft capsule), liquid formulations, injectables, suppositories, and sustained-release preparations by, for example, mixing Compound (I), (II), (III) or (IIIa) or(and) the above-mentioned concomitant drug with pharmacologically acceptable carriers according to a method known per se. The injectables can be administered intravenously, intramuscularly, subcutaneously or into organs or directly intralesionally.

As the pharmacologically acceptable carriers to be used for the production of the combined drug of the present invention, various organic or inorganic carriers employed conventionally as formulation materials are exemplified, and for example, an excipient, a lubricant, a binder, a disintegrating agent in solid formulations; and a solvent, a solubilizer, a suspending agent, an isotonic agent, a buffer, a soothing agent and the like in liquid formulations are exemplified. Further, if needed, usual additives such as a preservative, an antioxidant, a colorant, a sweetener, an adsorbent and a wetting agent may be used appropriately in an appropriate amount.

Examples of the excipient include lactose, white sugar, D-mannitol, starch, corn starch, crystalline cellulose, light anhydrous silicic acid, and the like.

Examples of the lubricant include magnesium stearate, potassium stearate, talc, colloidal silica, and the like.

Examples of the binder include crystalline cellulose, white sugar, D-mannitol, dextrin, hydroxypropyl cellulose, hydroxypropyl methylcellulose, polyvinylpyrrolidone, starch, sucrose, gelatin, methylcellulose, carboxymethylcellulose sodium, and the like.

Examples of the disintegrating agent include starch, carboxymethylcellulose, carboxymethylcellulose calcium, carboxymethylstarch sodium, L-hydroxypropyl cellulose, and the like.

Examples of the solvent include water for injection, alcohol, propylene glycol, macrogol, sesame oil, corn oil, olive oil, and the like.

Examples of the solubilizer include polyethylene glycol, propylene glycol, D-mannitol, benzyl benzoate, ethanol, trisaminomethane, cholesterol, triethanolamine, sodium carbonate, sodium citrate, and the like.

Examples of the suspending agent include surfactants such as stearyl triethanolamine, sodium laurylsulfate, laurylaminopropionic acid, lecithin, benzalkonium chloride, benzetonium chloride, glycerin monostearate; and hydrophilic polymers such as polyvinylalcohol, polyvinylpyrrolidone, carboxymethylcellulose sodium, methylcellulose, hydroxymethylcellulose, hydroxyethyl cellulose, hydroxypropyl cellulose, and the like.

Examples of the isotonic agent include glucose, D-sorbitol, sodium chloride, glycerin, D-mannitol, and the like.

Examples of the buffer include a buffer solution of phosphate, acetate, carbonate, citrate, and the like.

Examples of the soothing agent include benzylacohol, and the like.

Examples of the preservative include paraoxybenzoic esters, chlorobutanol, benzylalcohol, phenethylalcohol, dehydroacetic acid, sorbic acid, and the like.

Examples of the antioxidant include sulfite, ascorbic acid, α-tocopherol, and the like.

The compounding ratio of Compound (I), (II), (III) or (IIIa) and the concomitant drug in the combined drug of the present invention can be selected appropriately depending on the subject to be administered, administration route, disease to be treated, and the like.

For example, although the content of Compound (I), (II), (III) or (IIIa) in the combined drug of the present invention varies depending on the form of the preparation, it is usually about 0.01 to 100% by weight, preferably about 0.1 to 50% by weight, more preferably about 0.5 to 20% by weight, based on the total weight of the preparation.

Although the content of the concomitant drug in the combined drug of the present invention varies depending on the form of the preparation, it is usually about 0.01 to 100% by weight, preferably about 0.1 to 50% by weight, more preferably about 0.5 to 20% by weight, based on the total weight of the preparation.

Although the content of the additives such as carrier in the combined drug of the present invention varies depending on the form of the preparation, it is usually about 1 to 99.99% by weight, preferably about 10 to 90% by weight, based on the total weight of the preparation.

In addition, when Compound (I), (II), (III) or (IIIa) and the concomitant drug are formulated into preparations separately, the same contents may be employed.

These preparations can be produced by a per se known method employed conventionally in a formulation process.

For example, Compound (I), (II), (III) or (IIIa) or the concomitant drug can be made into an injectable by formulating as an aqueous injectable together with dispersants (e.g., Tween 80 (manufactured by Atlas Powder, USA), HCO 60 (manufactured by Nikko Chemicals Co., Ltd.), polyethylene glycol, carboxymethylcellulose, sodium alginate, hydroxypropyl methylcellulose, dextrin etc.), stabilizers (e.g. ascorbic acid, sodium pyrosulfite etc.), surfactants (e.g. Polysorbate 80, macrogol etc.), solubilizers (e.g. glycerin, ethanol etc.), buffers (e.g. phosphoric acid and alkali metal salt thereof, citric acid and alkali metal salt thereof etc.), isotonic agents (e.g. sodium chloride, potassium chloride, mannitol, sorbitol, glucose etc.), pH adjusting agents (e.g. hydrochloric acid, sodium hydroxide etc.), preservatives (e.g. ethyl paraoxybenzoate, benzoic acid, methyl paraoxybenzoate, propyl paraoxybenzoate, benzyl alcohol etc.), dissolving agents (e.g. concentrated glycerin, meglumine etc.), solubilizers (e.g. propylene glycol, white sugar etc.), soothing agents (e.g. glucose, benzyl alcohol etc.) and the like, or by formulating as an oil-soluble injectable by dissolving, suspending or emulsifying in a vegetable oil such as an olive oil, a sesame oil, a cottonseed oil and a corn oil or a solubilizer such as propylene glycol.

For preparing an oral preparation, for example, according to a method known per se, excipients (e.g. lactose, white sugar, starch etc.), disintegrating agents (e.g. starch, calcium carbonate etc.), binders (e.g. starch, gum arabic, carboxymethylcellulose, polyvinylpyrrolidone, hydroxypropyl cellulose etc.) or lubricants (e.g. talc, magnesium stearate, polyethylene glycol 6000 etc.) are added to Compound (I), (II), (III) or (IIIa) or the concomitant drug, and the resulting mixture is compressed to mold, if necessary, followed by coating according to a method known per se for the purpose of taste masking, enteric solubility or durability to obtain an oral preparation. As the coating agent, for example, hydroxypropyl methylcellulose, ethylcellulose, hydroxymethylcellulose, hydroxypropyl cellulose, polyoxyethylene glycol, Tween 80, Pluronic F68, cellulose acetate phthalate, hydroxypropyl methylcellulose phthalate, hydroxymethylcellulose acetate succinate, Eudragit (manufactured by Rohm, Germany, methacrylic acid/acrylic acid copolymer) and a pigment (e.g. bengala, titanium dioxide etc.) are used. The oral preparation may be a rapid-releasing preparation or a sustained-release preparation.

For preparing a suppository, for example, according to a method known per se, Compound (I), (II), (III) or (IIIa) or the concomitant drug can be formulated into an oily or aqueous solid, semisolid or liquid suppository. Examples of an oily base used for the above-mentioned composition include glyceride of higher fatty acid [e.g. cacao butter, witepsols (manufactured by Dynamite Nobel, Germany) etc.], medium fatty acid [e.g. mygliols (manufactured by Dynamite Nobel, Germany) etc.], and vegetable oil (e.g. sesame oil, soybean oil, cottonseed oil etc.). In addition, examples of an aqueous base include polyethylene glycols, and propylene glycol, and examples of an aqueous gel base include natural gums, cellulose derivatives, vinyl polymers, and acrylic acid polymers.

Examples of the above-mentioned sustained-release preparation include a sustained-release microcapsule preparation and the like.

For formulating into sustained-release microcapsules, a method known per se may be employed.

Compound (I), (II), (III) or (IIIa) is preferably formulated into an oral preparation such as solid preparations (e.g. powders, granules, tablets, capsules), or into a rectal preparation such as a suppository. An oral preparation is particularly preferable.

The concomitant drug can be made into the above-mentioned dosage forms depending on a kind of the drug.

The dose of the combined drug of the present invention varies depending on the kind of Compound (I), and an age, a weight, symptom, a dosage form, an administration method, an administration term, etc. and, for example, per patient (adult, body weight about 60 kg), it may usually be selected from a range of about 0.1 mg to about 500 mg, preferably from a range of about 1 mg to about 100 mg as the compound and concomitant drug of the present invention, respectively, in the case of oral administration; from a range of about 0.01 mg to about 100 mg, preferably from a range of about 0.1 mg to about 10 mg, respectively, in the case of parenteral administration. These dosage may be administered in once to 3 divided doses a day. Of course, since the dose varies under various conditions as described above, administration with a dose smaller than the above-mentioned dose is sufficient in some cases, and administration with a dose exceeding the above-mentioned range is needed in other cases.

As for the concomitant drug, any amount may be set in such a range that side effect is not problematic. The daily dosage as the concomitant drug varies depending on a degree of symptom, an age, sex, body weight and difference of sensitivity of the subject to be administered, time and interval of administration, nature, dispensation and kind of a pharmaceutical preparation, and a kind of an active ingredient, and it is, but is not particularly limited to, for example, usually about 0.001 to 2000 mg, preferably about 0.01 to 500 mg, further preferably about 0.1 to 100 mg per 1 kg body weight of a mammal as an amount of the drug in the case of oral administration, and this is usually administered in once to 3 divided doses a day.

When the drug of the present invention is administered, the compound of the present invention may be administered after the concomitant drug is administered first, or the concomitant drug may be administered after the compound of the present invention is administered first, although the compound of the present invention and the concomitant drug may be administered at the same time. When they are administered at a different time, the time difference varies depending on an active ingredient to be administered, a dosage form and an administration method, and, for example, when the concomitant drug is administered first, exemplified is a method of administering the compound of the present invention within 1 minute to 3 days, preferably within 10 minutes to 1 day, more preferably within 15 minutes to 1 hour after the administration of the concomitant drug. When the compound of the present invention is administered first, exemplified is a method of administering the concomitant drug within 1 minute to 1 day, preferably within 10 minutes to 6 hours, more preferably within 15 to 1 hour after the administration of the compound of the present invention.

As a preferable administration method, for example, about 0.001 to 200 mg/kg of the concomitant drug formulated into an oral preparation is orally administered, and after about 15 minutes, about 0.005 to 100 mg/kg of the compound of the present invention which has been formulated into an oral preparation is orally administered as one day amount.

### Examples

The methods for production and use of the present invention will be further explained by way of the following Example and Test Examples, but the present invention is not limited to these. Other embodiments which fall within the spirit and scope of the invention defined by the claims are included in the present invention.

### Test Example 1

Strategy for cloning of the cDNAs encoding the human CaR

Strategy for cloning of the cDNAs encoding the human CaR is shown below. To amplify the cDNA encoding the N-terminal moiety of the human CaR, the synthetic DNA primers, Ca1-U : 5'-AGAGTCGACGCCACCATGGCATTTTATAGCTGCTGCTGG-3' [SEQ ID NO: 1] and Ca1-L : 5'-AAATGAGCTCTCGGTTGGTGGCCTTGAC-3' [SEQ ID NO: 2], were constructed. In this case, SalI site was added at the 5' end of amplified cDNA. To amplify the cDNA encoding the C-terminal moiety of the human CaR, the synthetic DNA primers, Ca2-U : 5'-AAACGAGCTCTCCTACCTCCTCCTCTTC-3' [SEQ ID NO: 3] and Ca2-L : 5'-TCTGCGGCCGCTCCCTAGCCCAGTCTTCTCCTTCC-3' [SEQ ID NO: 4], were constructed. In this case, NotI site was added at the 3' end of amplified cDNA. PCR was carried out by Hot Start method. To the reaction solution of the upper phase was added 1 pg of the human kidney-derived cDNA (TOYOBO), 0.3 mM dNTPs and 2.5 unit LA Taq DNA polymerase (Takara shuzo co.) and filled up to 30 µl with water and buffer attached to the enzyme. To the reaction solution of the lower phase was added 12.5 µM each of the synthetic primers and 0.5 mM dNTPs and filled up to 20 µl with water and buffer attached to the enzyme. The reaction solution of the upper phase was added on the lower phase covered with an AmpliWax PCR Geum100 (Takara Shuzo Co.). The samples were subject to PCR amplification using a Terminal Cycler (Perkin-Elmer Co.). The amplified cDNAs were confirmed by agarose gel electrophoresis.

### Test Example 2

### Preparation of CaR-expression CHO cells

The PCR products obtained in Test Example 1 were separated by agarose gel electrophoresis. The PCR products were excised from the gel and purified, and subcloned into pT7Blue-T vector (Takara Shuzo Co.). The cDNA fragment encoding the N-terminal moiety of the human CaR was released from the subcloned pT7Blue-T vector by treating with SalI and SacI. The cDNA fragment encoding the C-terminal moiety of the human CaR was released from the subcloned pT7Blue-T vector by treating with SacI and NotI. Using DNA Ligation kit (Takara Shuzo Co.), these fragments were inserted between the site of SalI- and NotI- in the digested pMSRαneo vector. Thus, the pMSRαneo-CaR for animal cell expression was constructed.

Ten µg of the pMSRαneo-CaR was added to the solution containing 8x10⁶ CHO-K1 cells, and transfection was carried out using Gene Pulser (0.4cm cuvette, 0.25kV, 960mF) (Bio-Rad Laboratories). The cells were cultured in HamF12 containing 10% fetal calf serum for one day. After passage, the cells were cultured in HamF12 containing 10% fetal calf serum and 500µg/ml Genetisine. The cells were seeded on 96-well plate in 1x10³ cells/well and transformants, CaR-expressing CHO cells, were selected in the selection medium.

### Test Example 3

### Selection of the CaR-expressing CHO cell line by calcium mobilization assay

A method for calcium mobilization assay is shown below. The CaR-expressing CHO cells were seeded on a 96-well white plate in 2x10⁴ cells/well, followed by cultivation for 48 hours. After washing the cells with Phosphate-Buffered Saline, 100 µl of buffer solution (120 mM NaCl, 22 mM NaHCO₃, 6 mM KCl, 0.2 mM CaCl₂, 1 mM MaCl₂, 5 mM glucose, 5 mM HEPES (pH 7.4)) containing 5 µM FuraPE3AM (Texas Fluorescence Laboratories) was added to the wells and kept at 37°C for 1 hour. The cells were washed twice with Phosphate-Buffered Saline. After adding 180 µl of the reaction buffer solution (130 mM NaCl, 5.4 mM KCl, 0.2 mM CaCl₂, 0.9 mM MgCl₂, 10 mM glucose, 20 mM HEPES (pH 7.4)) to the wells, 20 µl of 60 mM CaCl₂ was added and intracellular calcium concentration were measured with a fluorometric imaging plate reader (FDSS 2000, Hamamatsu photonics). One clone increasing intracellular calcium concentration was selected and used for the following experiment.

### Test Example 4

### GTPγS binding assay

Preparation of membrane fraction is described bellow. The human CaR-expressing CHO cells were inoculated to a F500 flask in 1.8x10⁵ cells/flask followed by cultivation for 2 days. The cells were scraped with 10ml of Phosphate-Buffered Saline containing 0.02% EDTA. After centrifugation (2000 rpm, 10 min) of the cells, the cell pellet was resuspended into 12 ml of homogenate buffer solution (10 mM NaHCO₃, 1 mM EDTA, 1x Protease inhibitor cocktail (pH 7.4)) and homogenized by Polytron^{™} (2000 rpm, 1 min). The cell debris was removed by centrifugation (2000 rpm, 10 min), and then the CaR-expressing cell membrane fraction was collected by ultracentrifugation (Beckman 70 Ti type rotor, 30000 rpm, 1 hour).

The GTPγS binding activity was measured as follows. Twenty µg of the CaR-expressing cell membrane was incubated with test compounds for 10min. The assays were carried out at room temperature for an hour in a reaction mixture solution containing 20 mM HEPES (pH.7.4), 100 mM NaCl, 1 mM MgCl₂, 167 µg/ml DTT, 5 µM guanosine 5'-diphosphate, 0.4 nM [³⁵S]-guanosine 5'-(γ-thio) triphosphate ([³⁵S]-GTPγS) and 6 mM CaCl₂. The reaction mixture was filtered through a GF/C filter. After washing four times with 300 µl of Phosphate-Buffered Saline, the amount of [³⁵S]-GTPγS bound to the filter was measured using a Top-count scintillation counter.

Effects of test compounds on [³⁵S]-GTPγS binding were expressed in percentage terms. This was calculated from the equation [100x(t'-b)]/(t-b)]. Herein, t', t and b are values of [³⁵S]-GTPγS binding (dpm), t' is a value in the presence of 6mM calcium and the test compound, t is a value in the presence of 6mM calcium only and b is a value in the absence of both 6mM calcium and the test compound.

The antagonist dose-dependently decreased [³⁵S]-GTPγS binding in membrane preparation. The agonist dose-dependently increased [³⁵S]-GTPγS binding in membrane preparation.

### Test Compound 1:

N-(2-(4-methoxyphenyl)-2-phenylethyl)-5-phenyl-7-(trifluoromethyl)-4,5,6,7-tetrahydropyrazolo[1,5-a]pyrimidine-3-carboxamide

### Test Compound 2:

N-(bis(4-methoxyphenyl)methyl)-7-methyl-5-phenyl-4,5,6,7-tetrahydropyrazolo[1,5-a]pyrimidine-3-carboxamide

### Test Compound 3:

N-(1-ethyl-1-(4-(trifluoromethyl)phenyl)propyl)-5-phenyl-7-(trifluoromethyl)-4,5,6,7-tetrahydropyrazolo[1,5-a]pyrimidine-3-carboxamide

### Test Compound 4:

3-((4-(bis(4-methylphenyl)methoxy)-1-piperidinyl)carbonyl)-5-phenyl-7-(trifluoromethyl)-4,5,6,7-tetrahydropyrazolo[1,5-a]pyrimidine

### Test Compound 5:

N-(1-adamantyl)-7-methyl-5-phenyl-7-(trifluoromethyl)-4,5,6,7-tetrahydropy-razolo[1,5-a]pyrimidine-3-carboxamide

### Test Compound 6:

N-(1-ethyl-1-(4-(3-thienyl)phenyl)propyl)-7,7-dimethyl-5-phenyl-4,5,6,7-tetrahydropyrazolo[1,5-a]pyrimidine-3-carboxamide

### Test Compound 7:

5-phenyl-N-(2-phenyl-2-(1-pyrrolidinyl)ethyl)-7-(trifluoromethyl)-4,5,6,7-tetrahydropyrazolo[1,5-a]pyrimidine-3-carboxamide

The results are shown in Table 1.

**[Table 1]**

| Test Compound | [³⁵S]-GTPγS binding (%) |
|---|---|
| 1* | 34 (1µM) |
| 2* | 0 (1µm) |
| 3* | 0 (1µM) |
| 4* | 10 (1µm) |
| 5* | 12 (1µM) |
| 6* | 0 (1µm) |
| 7** | 256 (10µM) |

| | |
|---|---|
| * : antagonist ** : agonist | |

### Example 1

| | | |
|---|---|---|
| (1) | Test Compound 1 | 8.0 g |
| (2) | Active vitamin D3 | 8.0 g |
| (3) | Lactose | 60.0 g |
| (4) | Corn starch | 35.0 g |
| (5) | Gelatin | 3.0 g |
| (6) | Magnesium stearate | 2.0 g |

Using 10% by weight aqueous gelatin solution (30 ml, 3.0 g as gelatin), a mixture of Test Compound 1 (8.0 g), active vitamin D3 (8.0 g), lactose (60.0 g) and corn starch (35.0 g) is granulated by passing through a 1 mm mesh sieve, and the resulting granules are dried at 40°C and passed through the sieve again. The thus obtained granules are mixed with magnesium stearate (2.0 g), and are compressed. The resulting core tables are coated with a sugar film formed from an aqueous suspension of sucrose, titanium dioxide, talc and gum arabic. The coated tablets are polished with beewax to obtain 1,000 coated tablets.

### Industrial Applicability

The drug comprising a combination of Compound (I), (II), (III) or (IIIa) and a resorption inhibitor of the present invention has an excellent calcium receptor modulating action and enhances the secretion of PTH, thus it is useful as drugs for treating bone diseases, kidney-acting drugs, central nervous system and endocrine-acting drugs, digestive system-acting drugs, and the like.

SEQUENCE LISTING

<110> TAKEDA PHARMACEUTICAL COMPANY LIMITED

<120> PHARMACEUTICAL COMBINATION

<130> MUB08-3414EP GD/LM

<150> JP 2006-62768

<151> 2006-03-08

<160> 4

<170> PatentIn version 3.3

<210> 1
<211> 39
<212> DNA
<213> Artificial Sequence
<220> primer
<400> 1
agagtcgacg ccaccatggc attttatagc tgctgctgg 39

<210> 2
<211> 28
<212> DNA
<213> Artificial Sequence
<220> primer
<400> 2
aaatgagctc tcggttggtg gccttgac 28

<210> 3
<211> 28
<212> DNA
<213> Artificial Sequence
<220> primer
<400> 3
aaacgagctc tcctacctcc tcctcttc 28

<210> 4
<211> 35
<212> DNA
<213> Artificial Sequence
<220> primer
22<400> 4
tctgcggccg ctccctagcc cagtcttctc cttcc 35

## Claims

1. A drug comprising a combination of a calcium receptor modulator and a bone resorption inhibitor, wherein the calcium receptor modulator comprises a compound represented by the formula (I): 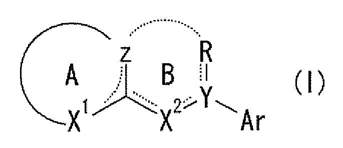 wherein ring A is an optionally substituted 5- to 7-membered ring;
ring B is an optionally substituted 5- to 7-membered heterocyclic ring;
X¹ is CR¹ (wherein R¹ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- is -CO-, -CS-, - SO- or -SO₂-, and Z² is an optionally substituted hydrocarbon group, an optionally substituted heterocyclic group, an optionally substituted hydroxyl group, or an optionally substituted amino group)), CR¹R² (wherein R¹ is as defined above, R² is a hydrogen or an optionally substituted hydrocarbon group), N or NR¹³ (wherein R¹³ is a hydrogen, an optionally substituted hydrocarbon group, an optionally substituted hydroxyl group, an optionally substituted amino group, cyano group, a halogen atom, an optionally substituted heterocyclic group, or a group of the formula: -Z¹-Z² (wherein -Z¹- and Z² are as defined above));
X² is N or NR³ (wherein R³ is a hydrogen, an optionally substituted hydrocarbon group, an optionally substituted hydroxyl group, an optionally substituted amino group, cyano group, a halogen atom, an optionally substituted heterocyclic group, or a group of the formula: -Z¹-Z² (wherein -Z¹- and Z² are as defined above);
Y is C, CR⁴ (wherein R⁴ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above)) or N;
Z is CR⁵ (wherein R⁵ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above)), CR⁵R⁶ (wherein, R⁵ is as defined above and R⁶ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above), and R⁵ and R⁶ may be the same or different), N or NR⁷ (wherein R⁷ is a hydrogen, an optionally substituted hydrocarbon group, an optionally substituted hydroxyl group, an optionally substituted amino group, cyano group, a halogen atom, an optionally substituted heterocyclic group, or a group of the formula: -Z¹-Z² (wherein -Z¹- and Z² are as defined above));
Ar is an optionally substituted cyclic group;
R is a hydrogen, an optionally substituted hydrocarbon group, an optionally substituted hydroxyl group, an optionally substituted amino group, an optionally substituted thiol group, an optionally substituted sulfonyl group or an optionally substituted sulfinyl group, or R and Z may be combined to form ring B; and
----- is a single bond or a double bond;
or a salt thereof or a prodrug thereof.

2. A drug comprising a combination of a calcium receptor modulator and a bone resorption inhibitor, wherein the calcium receptor modulator comprises a compound represented by the formula (II): 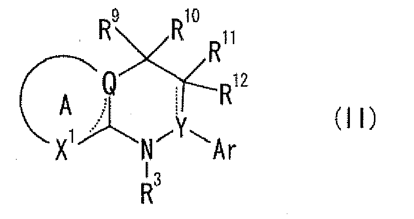 wherein ring A is an optionally substituted 5- to 7-membered ring;
Q is C, CR⁵ (wherein, R⁵ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula : -Z¹-Z² (wherein -Z¹- is -CO-, -CS-, - SO- or -SO₂-, and Z² is an optionally substituted hydrocarbon group, an optionally substituted heterocyclic group, an optionally substituted hydroxyl group, or an optionally substituted amino group)), or N;
X¹ is CR¹ (wherein R¹ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above)), CR¹R² (wherein R¹ is as defined above, and R² is a hydrogen, or an optionally substituted hydrocarbon group), N, or NR¹³ (wherein, R¹³ is a hydrogen, an optionally substituted hydrocarbon group, an optionally substituted hydroxyl group, an optionally substituted amino group, an optionally substituted heterocyclic group, or a group of the formula: -Z¹-Z² (wherein -Z¹- and Z² are as defined above));
R³ is a hydrogen, an optionally substituted hydrocarbon group, an optionally substituted hydroxyl group, an optionally substituted amino group, an optionally substituted heterocyclic group, or a group of the formula: -Z¹-Z² (wherein -Z¹- and Z² are as defined above);
Y is C, CR⁴ (wherein, R⁴ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above)), or N;
Ar is an optionally substituted cyclic group;
R⁹ and R¹⁰ are the same or different and are a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above); and
R¹¹ and R¹² are the same or different and are a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z^{1'} -Z² (wherein, -Z^{1'}- is -CS-, -SO- or -SO₂-, and Z² is as defined above); or R⁹ and R¹⁰, or R¹¹ and R¹² may be combined to form an oxo group, methylene group or a ring; or R¹⁰ and R¹¹ may be combined to form a ring; and
----- is a single bond or a double bond;
provided that
(1) when ring A is a 6-membered ring and Q is C or CR⁵, X¹ is C-Z¹-Z², C(-Z¹-Z²)R² or N-Z¹-Z², and neither R⁹ nor R¹⁰ is a hydrogen, or R⁹ and R¹⁰ are not combined to form an oxo group, or R¹⁰ and R¹¹ are not combined to form a 5-membered ring,
(2) when ring A is a 6-membered ring and Q is N, X¹ is C-Z¹-Z², C(-Z¹-Z²)R² or N-Z¹-Z², and R⁹ and R¹⁰ are not combined to form an oxo group,
(3) when ring A is a 5-membered ring and Q is C or CR⁵, X¹ is C-Z¹-Z², C(-Z¹-Z²)R² or N-Z¹-Z², and Z² is an optionally substituted amino group, and
(4) when ring A is a 5-membered ring and Q is N, at least one of R⁹ and R¹⁰ is CHR¹⁵R¹⁶ (wherein, at least one of R¹⁵ and R¹⁶ are the same or different and are a hydrogen, an optionally substituted hydrocarbon group, an optionally substituted hydroxyl group, an optionally substituted amino group, an optionally substituted thiol group, a halogen atom, an optionally substituted heterocyclic group, or a group of the formula: -Z¹-Z² (wherein, -Z¹- and Z² are as defined above)) and the other is other than an optionally substituted phenyl group; or a salt thereof or a prodrug thereof.

3. A drug comprising a combination of a calcium receptor modulator and a bone resorption inhibitor, wherein the calcium receptor modulator comprises a compound represented by the formula (III): 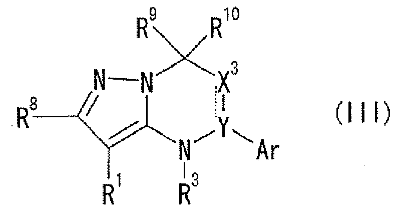 wherein R¹ is a hydrogen, an optionally substituted hydrocarbon group, an optionally substituted hydroxyl group, an optionally substituted thiol group, an optionally substituted amino group, cyano group, a halogen atom, an optionally substituted heterocyclic group, or a group of the formula: -Z¹-Z² (wherein -Z¹- is -CO-, -CS-, -SO- or - SO₂-, and Z² is an optionally substituted hydrocarbon group, an optionally substituted heterocyclic group, an optionally substituted hydroxyl group, or an optionally substituted amino group);
R³ is a hydrogen, an optionally substituted hydrocarbon group, an optionally substituted hydroxyl group, an optionally substituted amino group, an optionally substituted heterocyclic group, or a group of the formula: -Z¹-Z² (wherein -Z¹- and Z² are as defined above);
Y is C, CR⁴ (wherein, R⁴ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above)) or N;
R⁸ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above);
Ar is an optionally substituted cyclic group;
R⁹ and R¹⁰ are the same or different and are a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above), or R⁹ and R¹⁰ may be combined to form an oxo group, methylene group or a ring;
X³ is a bond, oxygen atom, an optionally oxidized sulfur atom, N, NR^{7'} (wherein, R^{7'} is a hydrogen, an optionally substituted hydrocarbon group, an optionally substituted hydroxyl group, an optionally substituted amino group, an optionally substituted heterocyclic group, or a group of the formula -Z^{1'}-Z² (wherein -Z^{1'}- is -CS-, -SO- or -SO₂-, and Z² is as defined above)), or an optionally substituted bivalent C₁₋₂ hydrocarbon group; and
----- is a single bond or a double bond;
provided that at least one of R⁹ and R¹⁰ is CHR¹⁵R¹⁶ (wherein, R¹⁵ and R¹⁶ are the same or different and are a hydrogen, an optionally substituted hydrocarbon group, an optionally substituted hydroxyl group, an optionally substituted amino group, an optionally substituted thiol group, a halogen atom, an optionally substituted heterocyclic group, or a group of the formula: -Z¹-Z² (wherein -Z¹- and Z² are as defined above)) and the other is other than an optionally substituted phenyl group; or a salt thereof or a prodrug thereof.

4. A drug comprising a combination of a calcium receptor modulator and a bone resorption inhibitor, wherein the calcium receptor modulator comprises a compound represented by the formula (IIIa): 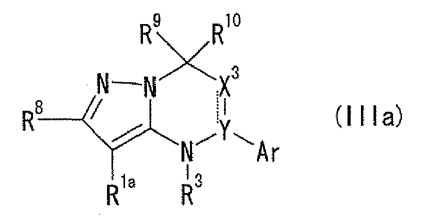 wherein, R^{1a} is (1) an optionally substituted heterocyclic group, or (2) a group of the formula: -Z^{1a}-Z^{2a} (wherein -Z^{1a}-is -CO-, -CS-, -SO- or -SO₂-, and Z^{2a} is (i) an optionally substituted heterocyclic group, (ii) -NR^{20a}(CR^{21a}R^{22a}R^{23a}) (wherein, (a) R^{20a} is a hydrogen or an optionally substituted hydrocarbon group; and R^{21a} is an optionally substituted heterocyclic group which may be fused with an optionally substituted benzene ring, or an optionally substituted phenyl group which may be fused with an optionally substituted aromatic heterocyclic ring, and R^{22a} and R^{23a} are the same or different and are an optionally substituted hydrocarbon group or an optionally substituted heterocyclic group or R^{22a} and R^{23a} may be combined to form a ring, or (b) R^{20a} is a hydrogen or an optionally substituted hydrocarbon group; and R^{21a}, R^{22a} and R^{23a} are the same or different and are an optionally substituted C₁₋₈ aliphatic hydrocarbon group, provided that the sum total of the carbon atoms is 7 or more), (iii) -NR^{20a}R^{25a} (wherein, R^{20a} is as defined above and R^{25a} is an optionally substituted C₆₋₁₀ aryl-C₂₋₄ alkyl, C₆₋₁₀ aryloxy-C₂₋₄ alkyl, C₆₋₁₀ arylamino-C₂₋₄ alkyl, C₇₋₁₄ aralkylamino-C₂₋₄ alkyl, heterocyclic ring-C₂₋₄ alkyl or heterocyclic group), (iv) a substituted 5- to 7-membered cyclic amino group, or (v) -OR^{24a} (wherein, R^{24a} is
(a) an optionally substituted C₇₋₁₄ aralkyl group,
(b) an optionally substituted C₃₋₇ alicyclic hydrocarbon group,
(c) an optionally substituted C₇₋₂₄ aliphatic hydrocarbon group, or
(d) an optionally substituted heterocyclic group);
R³ is a hydrogen, an optionally substituted hydrocarbon group, an optionally substituted hydroxyl group, an optionally substituted amino group, an optionally substituted heterocyclic group, or a group of the formula: -Z¹-Z²(wherein -Z¹- is -CO-, -CS-, -SO- or -SO₂-, and Z² is an optionally substituted hydrocarbon group, an optionally substituted heterocyclic group, an optionally substituted hydroxyl group, or an optionally substituted amino group);
Y is C, CR⁴ (wherein R⁴ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above)) or N;
R⁸ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above);
Ar is an optionally substituted cyclic group;
R⁹ and R¹⁰ are the same or different and are a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above), or R⁹ and R¹⁰ may be combined to form an oxo group, methylene group or a ring;
X³ is a bond, oxygen atom, an optionally oxidized sulfur atom, N, NR^{7'} (wherein R^{7'} is a hydrogen, an optionally substituted hydrocarbon group, an optionally substituted hydroxyl group, an optionally substituted amino group, an optionally substituted heterocyclic group, or a group of the formula -Z¹-Z² (wherein, -Z^{1'}- is -CS-, -SO- or -SO₂-, and Z² is as defined above)), or an optionally substituted bivalent C₁₋₂ hydrocarbon group; and
----- is a single bond or a double bond;
provided that at least one of R⁹ and R¹⁰ is CHR¹⁵R¹⁶ (wherein R¹⁵ and R¹⁶ are the same or different and are a hydrogen, an optionally substituted hydrocarbon group, an optionally substituted hydroxyl group, an optionally substituted amino group, an optionally substituted thiol group, a halogen atom, an optionally substituted heterocyclic group, or a group of the formula:-Z¹-Z² (wherein -Z¹- and Z² are as defined above)) and the other is other than an optionally substituted phenyl group; or a salt thereof or a prodrug thereof.

5. The drug comprising the combination according to any one of claims 1 to 4, wherein the bone resorption inhibitor is one or more medicines selected from the group consisting of (1) estrogen, (2) selective estrogen receptor modulators (SERM), (3) RANKL inhibitors, (4) strontium, (5) active vitamin D3, (6) vitamin K2, (7) ipriflavone preparations, (8) vitronectin receptor antagonists, (9) V-H+-ATPase inhibitors, (10) Src kinase inhibitors and (11) cathepsin K inhibitors.

6. The drug comprising the combination according to any one of claims 1 to 4, which is an agent for preventing or treating diseases caused by abnormality of calcium concentration or a calcium receptor in living body.

7. The drug comprising the combination according to any one of claims 1 to 4, which is an agent for preventing or treating bone diseases.

8. The drug comprising the combination according to any one of claims 1 to 4, which is an agent for preventing or treating osteoporosis or fracture.

9. A method for preventing or treating diseases caused by abnormality of calcium concentration or a calcium receptor in living body which comprises administering to a mammal an effective amount of a calcium receptor modulator and an effective amount of a bone resorption inhibitor, wherein the calcium receptor modulator comprises a compound represented by the formula (I): 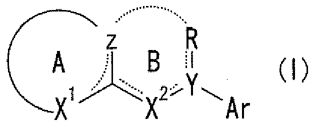 wherein ring A is an optionally substituted 5- to 7-membered ring;
ring B is an optionally substituted 5- to 7-membered heterocyclic ring;
X¹ is CR¹ (wherein R¹ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein, -Z¹- is -CO-, -CS-, - SO- or -SO₂-, and Z² is an optionally substituted hydrocarbon group, an optionally substituted heterocyclic group, an optionally substituted hydroxyl group, or an optionally substituted amino group)), CR¹R² (wherein, R¹ is as defined above, R² is a hydrogen or an optionally substituted hydrocarbon group), N or NR¹³ (wherein, R¹³ is a hydrogen, an optionally substituted hydrocarbon group, an optionally substituted hydroxyl group, an optionally substituted amino group, cyano group, a halogen atom, an optionally substituted heterocyclic group, or a group of the formula: -Z¹-Z² (wherein, -Z¹- and Z² are as defined above));
X² is N or NR³ (wherein, R³ is a hydrogen, an optionally substituted hydrocarbon group, an optionally substituted hydroxyl group, an optionally substituted amino group, cyano group, a halogen atom, an optionally substituted heterocyclic group, or a group of the formula: -Z¹-Z² (wherein -Z¹- and Z² are as defined above);
Y is C, CR⁴ (wherein, R⁴ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein -Z¹- and Z² are as defined above)) or N;
Z is CR⁵ (wherein, R⁵ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein, -Z¹- and Z² are as defined above)), CR⁵R⁶ (wherein, R⁵ is as defined above and R⁶ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein, -Z¹- and Z² are as defined above), and R⁵ and R⁶ may be the same or different), N or NR⁷ (wherein, R⁷ is a hydrogen, an optionally substituted hydrocarbon group, an optionally substituted hydroxyl group, an optionally substituted amino group, cyano group, a halogen atom, an optionally substituted heterocyclic group, or a group of the formula: -Z¹-Z² (wherein, -Z¹- and Z² are as defined above));
Ar is an optionally substituted cyclic group;
R is a hydrogen, an optionally substituted hydrocarbon group, an optionally substituted hydroxyl group, an optionally substituted amino group, an optionally substituted thiol group, an optionally substituted sulfonyl group or an optionally substituted sulfinyl group, or R and Z may be combined to form ring B; and
----- is a single bond or a double bond; or a salt thereof or a prodrug thereof.

10. Use of a calcium receptor modulator and a bone resorption inhibitor for manufacturing a drug for preventing or treating diseases caused by abnormality of calcium concentration or a calcium receptor in living body, wherein the calcium receptor modulator is a compound represented by the formula (I): 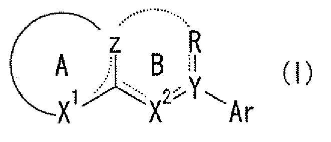 wherein ring A is an optionally substituted 5- to 7-membered ring;
ring B is an optionally substituted 5- to 7-membered heterocyclic ring;
X¹ is CR¹ (wherein, R¹ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein, -Z¹- is -CO-, -CS-, - SO- or -SO₂-, and Z² is an optionally substituted hydrocarbon group, an optionally substituted heterocyclic group, an optionally substituted hydroxyl group, or an optionally substituted amino group)), CR¹R² (wherein, R¹ is as defined above, R² is a hydrogen or an optionally substituted hydrocarbon group), N or NR¹³ (wherein, R¹³ is a hydrogen, an optionally substituted hydrocarbon group, an optionally substituted hydroxyl group, an optionally substituted amino group, cyano group, a halogen atom, an optionally substituted heterocyclic group, or a group of the formula: -Z¹-Z² (wherein -Z¹- and Z² are as defined above));
X² is N or NR³ (wherein, R³ is a hydrogen, an optionally substituted hydrocarbon group, an optionally substituted hydroxyl group, an optionally substituted amino group, cyano group, a halogen atom, an optionally substituted heterocyclic group, or a group of the formula: -Z¹-Z² (wherein -Z¹- and Z² are as defined above);
Y is C, CR⁴ (wherein, R⁴ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein, -Z¹- and Z² are as defined above)) or N;
Z is CR⁵ (wherein, R⁵ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein, -Z¹- and Z² are as defined above)), CR⁵R⁶ (wherein, R⁵ is as defined above and R⁶ is a hydrogen, an optionally substituted hydrocarbon group, an optionally substituted hydroxyl group, an optionally substituted amino group, an optionally substituted thiol group, cyano group, a halogen atom, an optionally substituted heterocyclic group, or a group of the formula: -Z¹-Z² (wherein, -Z¹- and Z² are as defined above), and R⁵ and R⁶ may be the same or different), N or NR⁷ (wherein, R⁷ is a hydrogen, an optionally substituted hydrocarbon group, an optionally substituted hydroxyl group, an optionally substituted amino group, cyano group, a halogen atom, an optionally substituted heterocyclic group, or a group of the formula: -Z¹-Z² (wherein, -Z¹- and Z² are as defined above));
Ar is an optionally substituted cyclic group;
R is a hydrogen, an optionally substituted hydrocarbon group, an optionally substituted hydroxyl group, an optionally substituted amino group, an optionally substituted thiol group, an optionally substituted sulfonyl group or an optionally substituted sulfinyl group, or R and Z may be combined to form ring B; and
----- is a single bond or a double bond; or a salt thereof or a prodrug thereof.
